# EUROPEAN PATENT SPECIFICATION

(11) **EP 2 185 294 B1**
(45) Date of publication and mention of the grant of the patent: **04.07.2018**
(21) Application number: 08796836.8
(22) Date of filing: 30.07.2008
(51) Int. Cl.: B05D 3/00

(54) **REDUCING TEMPLATE WITH COATING RECEPTACLE CONTAINING A MEDICAL DEVICE TO BE COATED**
REDUKTIONSSCHABLONE MIT BESCHICHTUNGSGEFÄSS MIT EINER ZU BESCHICHTENDEN MEDIZINISCHEN VORRICHTUNG
RÉDUCTION D'UN GABARIT AVEC REVÊTEMENT D'UN RÉCEPTACLE CONTENANT UN DISPOSITIF MÉDICAL À REVÊTIR

(30) Priority: 30.07.2007 US 962557 P
(43) Date of publication of application: 19.05.2010
(73) Proprietor: Atrium Medical Corporation, Hudson, NH 03051 (US)
(72) Inventor: HERWECK, Steve, A., Nashua, NH 03062 (US); KARWOSKI, Theodore, Hollis, NH 03049 (US); MARTAKOS, Paul, Pelham, NH 03076 (US); CORBELL, Scott, E., Litchfield, NH 03052 (US); LABRECQUE, Roger, Londonderry, NH 03053 (US); CONROY, Suzanne, Dracut, MA 01826 (US); SUNTER, Brian, Londonderry, NH 03053 (US); BROMANDER, Edward, Tewksbury, MA 01876 (US)
(74) Representative: Helbig, Christian
(86) International application number: PCT/US2008/071547
(87) International publication number: WO 2009/018313

(56) References cited:
- EP-A1- 0 650 823
- EP-A1- 0 653 300
- NL-A- 7 203 476
- US-A- 4 664 114
- US-A- 4 968 302
- US-A1- 2003 003 239
- US-A1- 2003 065 292
- US-A1- 2006 240 069
- US-A1- 2006 240 069
- US-B1- 6 753 071

## Description

### RELATED APPLICATIONS

The present application claims priority to and the benefit of United States Provisional Application No. 60/962557, filed July 30, 2007. The present application is also related to, and claims the benefit of, pending United States Application No. 11/238554, filed September 28, 2005 which claimed priority to, and the benefit of, United States Provisional Application No. 60/613745, filed September 28, 2004.

### FIELD OF THE INVENTION

The present invention relates to devices and techniques for storing medical devices to be coated, and regulating coatings on those medical devices prior to use. More specifically, the present invention is directed to apparatuses and techniques for storing medical devices, such as stents, balloons, and catheters, which require a coating prior to use, and providing a device or system of regulating a coating on the device prior to use. The coatings can be used for delivery of one or more biologically active agents, providing controlled short or long term release of biologically active components from the surface of the medical device, or can otherwise provide different chemical or physical characteristics to the device as coated.

### BACKGROUND OF THE INVENTION

Therapeutic agents may be delivered to a targeted location in a human utilizing a number of different methods. For example, agents may be delivered nasally, transdermally, intravenously, orally, or via other conventional methods. Delivery may vary by release rate (e.g., quick release, slow release, or biphasic release). Delivery may also vary as to how the drug is administered. Specifically, a drug may be administered locally to a targeted area, or administered systemically.

With systemic administration, the therapeutic agent is administered in one of a number of different ways including orally, inhalationally, or intravenously to be systemically processed by the patient. However, there are drawbacks to systemic delivery of a therapeutic agent, one of which is that high concentrations of the therapeutic agent travel to all portions of the patient's body and can have undesired effects at areas not targeted for treatment by the therapeutic agent. Furthermore, large doses of the therapeutic agent only amplify the undesired effects at non-target areas. As a result, the amount of therapeutic agent that results in application to a specific targeted location in a patient may have to be reduced when administered systemically to reduce complications from toxicity resulting from a higher dosage of the therapeutic agent.

An alternative to the systemic administration of a therapeutic agent is the use of a targeted local therapeutic agent delivery approach. With local delivery of a therapeutic agent, the therapeutic agent is administered using a medical device or apparatus, directly by hand, or sprayed on the tissue, at a selected targeted tissue location of the patient that requires treatment. The therapeutic agent emits, or is otherwise delivered, from the medical device apparatus, and/or carrier, and is applied to the targeted tissue location. The local delivery of a therapeutic agent enables a more concentrated and higher quantity of therapeutic agent to be delivered directly at the targeted tissue location, minimizing or eliminating broader systemic side effects. With local delivery, the therapeutic agent that escapes the targeted tissue location dilutes as it travels to the remainder of the patient's body, substantially reducing or eliminating systemic effects.

Local delivery is often carried out using a medical device as the delivery vehicle. One example of a medical device that is used as a delivery vehicle is a stent. Boston Scientific Corporation sells the Taxus® stent, which contains a polymeric coating for delivering Paclitaxel. Johnson & Johnson, Inc. sells the Cypher® stent which includes a polymeric coating for delivery of Sirolimus.

U.S. Patent Application No. US 2006/240069 A1 (hereinafter "Utas") describes a catheter assembly with bactericidal effect. Utas also describes a method for producing a catheter assembly, which comprises the steps of (a) providing a receptacle, (b) providing a hydrophilic catheter, (c) providing a wetting fluid, and (d) arranging at least an insertable part of the catheter in the receptacle and arranging the wetting fluid as a part of the catheter assembly, wherein at least one of the wetting fluid and the catheter further includes at least one salt of organic acid(s), and preferably a benzoate or a sorbate, and a pH buffer, for making the hydrophilic coating antimicrobial when wetted with the wetting fluid. The subject matter as claimed in this application is neither disclosed nor suggested.

In applying coatings to medical devices, such as stents and catheters, coverage and uniformity are important factors in getting optimal performance out of the coated medical device. If a device does not have the desired coverage then there may be areas on the device that do not have proper coating which can lead to problems. Similar problems can arise when the coating is not uniform. Non-uniform coatings can cause inconsistent interactions, especially when a therapeutic agent is being delivered. Ideally, the coating should be uniform over the desired portions of the medical device so that dosage and interaction with tissue can be better controlled.

Degradation of coating materials, and the therapeutic agents that can be included in coating materials, is a significant concern in the area of coated medical devices. Multiple strategies have been employed to prevent degradation of coating materials. An outer layer of porous biocompatible polymer covering the therapeutic coating layer has been used to control the release of the active agent and to reduce degradation of the therapeutic coating layer. The curing of coating materials by applying heat, UV light, chemical cross-linker, and/or reactive gas has also been used to reduce degradation of the coating. Unfortunately, curing a coating can reduce its therapeutic effectiveness.

In both of the aforementioned techniques, the coating material is deposited onto the medical device long before the device is implanted into the patient. Normally, the coated device would be manufactured, packaged, and then sent to another location and stored before use. The aforementioned techniques were designed to preserve the coating material already deposited on the medical device for the long period of time between when the device is coated and when the device is implanted (typically a week to multiple months). Preserving a coating material that is already applied to a device is difficult, in part, because the thin coating layer provides a large surface area for interaction with the surrounding environment and because oxygen, and other elements that may cause degradation, only need to diffuse a short distance through the thickness of the coating to reach all of the coating material.

A need exists for an apparatus configured to store and/or preserve a coating material and configured with a reducing template to form a substantially uniform coating of the coating material on a medical device in a predictable and repeatable manner shortly before the medical device is inserted or implanted into a patient.

### SUMMARY OF THE INVENTION

In accordance with the present invention an apparatus and a method for applying a coating to a medical device such as a stent, balloon, or catheter, shortly before insertion or implantation are provided that produce uniform consistent coverage in a predictable, repeatable and controllable manner and reduce the need for preservative components in the coating or for excessive curing or hardening of the coating.

An illustrative embodiment of the present invention includes an apparatus for coating a medical device. The apparatus includes a sealed receptacle that contains and preserves a coating material. The sealed receptacle has a proximal end and a distal end. The apparatus can further include a reducing template, as a portion of the sealed receptacle, or as a separate component coupled with the sealed receptacle during use. The reducing template regulates the application of the coating to the medical device as the device is withdrawn for use. The apparatus also includes the medical device disposed and sealed within the sealed receptacle and immersed in the coating material. The reducing template is adapted to wipe excess coating material from the medical device. An area defined by a cross-sectional inner profile of the reducing template is greater than an area defined by an outer profile of the medical device by a predetermined amount forming a gap area, as the medical device travels through the reducing template. The predetermined amount forming the gap area is determined at least in part by a thickness of coating material desired to remain on the medical device subsequent to movement of the medical device through the reducing template and out of the receptacle, wiping off excess coating material.

According to aspects of the present invention the reducing template can be coupled with the sealed receptacle. The reducing template can be disposed external to the sealed receptacle. Alternately, the sealed receptacle can include the reducing template. The reducing template can be disposed within the sealed receptacle.

According to other aspects of the present invention the sealed receptacle can include a proximal seal disposed at the proximal end of the sealed receptacle. The sealed receptacle can further include a proximal end cover disposed at a proximal end of the sealed receptacle. The sealed receptacle can include a distal seal disposed at the distal end of the sealed receptacle. The sealed receptacle can include a sleeve coupled with the proximal end of the sealed receptacle in a slidable manner. The reducing template can be disposed within the sleeve. The sleeve and the proximal end can be configurable relative to each other with one or more detents in a pre-use configuration, in an activation configuration, or both. The sleeve and the proximal end can be slidable toward each other from the pre-use configuration to the activation configuration. The sleeve can further include a seal breaching mechanism configured to breach the proximal seal while the proximal end and the sleeve are disposed in the activation configuration.

According to one aspect of the present invention, the sealed receptacle can include a catheter cap disposed within the sealed receptacle and coupled with the sealed receptacle at the distal end. The catheter cap can be permanently fixed to the distal end of the sealed receptacle by mechanical means, chemical means, thermal means, or any combination thereof.

According to other aspects of the present invention, the apparatus can further include a stylet partially disposed within the sealed receptacle, coupled with the sealed receptacle at the distal end, and protruding from the sealed receptacle through the proximal end. The stylet can be permanently fixed to the distal end of the sealed receptacle by mechanical means, chemical means, thermal means, or any combination thereof. The sealed receptacle can include a receptacle wall, and the receptacle wall can include a catheter cap disposed at the distal end of the sealed receptacle, a stylet disposed at the distal end of the sealed receptacle, or both. The sealed receptacle can include an end cap disposed at the distal end of the sealed receptacle and in contact with the receptacle wall. The end cap can include a catheter cap, a stylet, or both.

According to aspects of the present invention, the medical device can be mounted on a catheter. The catheter shaft can protrude from the sealed receptacle through the proximal end. According to other aspects of the present invention, the cross-sectional inner profile of the reducing template can be substantially circular or substantially elliptical in shape. The cross-sectional inner profile of the reducing template can be substantially polygonal or substantially irregular in shape.

According to another aspect of the present invention, the coating material can include a bio-absorbable liquid. The coating material can include a bio-absorbable liquid and at least one therapeutic agent. The coating material can include an oil containing at least one form of lipid. The coating material can include an oil containing at least one form of essential fatty acid. The coating material can include a partially cured oil. According to a different aspect of the present invention the medical device can include at least one device selected from the group consisting of: a stent, a catheter, a graft, and a balloon.

According to another aspect of the present invention the apparatus can also include an outer container, wherein the sealed receptacle is disposed within the outer container and the outer container is adapted to preserve the sterility of the sealed receptacle until use. The apparatus may also include inert gas disposed within the outer container to preserve the coating material.

Another illustrative embodiment of the present invention is a method for using an apparatus to coat a medical device with a coating material. The method includes providing the apparatus. The apparatus has a sealed receptacle having a proximal end and a distal end. The receptacle contains and optionally preserves (when applicable) a coating material. The apparatus also has a medical device that has an outer profile and that is disposed and sealed within the sealed receptacle and immersed in the coating material. The apparatus has a reducing template that has a cross-sectional inner profile. The reducing template is adapted to wipe excess coating material from the medical device. An area defined by the cross-sectional inner profile of the reducing template is greater than an area defined by the outer profile of the medical device by a predetermined amount forming a gap area. The predetermined amount forming the gap area is determined at least in part by a thickness of coating material desired to remain on the medical device subsequent to movement of the medical device through the reducing template and out of the sealed receptacle, wiping off excess coating material.

The method also includes altering a proximal end of the sealed receptacle to breach the sealed receptacle allowing the medical device to be withdrawn through the reducing template. The method further includes passing the medical device through the reducing template, wiping off excess coating material, resulting in a coating of predetermined thickness on the medical device. The method also includes altering a proximal end of the sealed receptacle allowing the medical device to be withdrawn through the reducing template, and withdrawing the medical device from the receptacle through the reducing template resulting in a coating of predetermined thickness on the medical device.

According to aspects of the invention, altering a proximal end of the sealed receptacle may include one or both of: changing the physical position of the proximal seal and removing the proximal seal from the apparatus. Altering a proximal end of the sealed receptacle can include sliding the sleeve toward the proximal end to activate the apparatus. Altering a proximal end of the sealed receptacle can include sliding the sleeve relative to the proximal end until the apparatus is disposed in the activation configuration breaching the proximal seal. Altering a proximal end of the sealed receptacle can include one or both of: changing the physical position of the proximal seal and removing the proximal seal from the apparatus.

In accordance with one embodiment of the present invention, a method of coating a medical device includes providing a storing and coating apparatus. The storing and coating apparatus includes a sealed receptacle having a proximal end and a distal end, the receptacle containing a coating material. The medical device can have an outer profile. The medical device can be disposed and sealed within the sealed receptacle and immersed in the coating material, the sealed receptacle configured to be unsealed or opened to enable the medical device to pass through at the time of use. The apparatus further including a reducing template having a cross-sectional inner profile, and adapted to receive the medical device and wipe excess coating material from the medical device. Wherein an area defined by the cross-sectional inner profile of the reducing template is greater than an area defined by the outer profile of the medical device by a predetermined amount forming a gap area; and wherein the predetermined amount forming the gap area is determined at least in part by a thickness of coating material desired to remain on the medical device subsequent to movement of the medical device out of the sealed receptacle and through the reducing template, wiping off excess coating material. The method continuing with withdrawing the medical device through the reducing template causing the reducing template to regulate a thickness of the coating formed on the medical device by wiping excess coating material from the device.

In accordance with various aspects of the present invention, the method can further include unsealing or opening the sealed receptacle prior to withdrawing the medical device through the reducing template. The act of withdrawing the medical device through the reducing template can cause an unsealing or opening of the sealed receptacle. The step of withdrawing the medical device includes the medical device can break open the sealed receptacle.

In accordance with one embodiment of the present invention, a kit for coating a medical device includes a coating material, the medical device, and a dispenser. The dispenser includes a sealed receptacle containing the coating material and the medical device, and a reducing template having a cross-sectional inner profile. The reducing template can be adapted to receive the medical device and wipe excess of the coating material from the medical device. An area defined by the cross-sectional inner profile of the reducing template is greater than an area defined by the outer profile of the medical device by a predetermined amount forming a gap area. In addition, the predetermined amount forming the gap area is determined at least in part by a thickness of the coating material desired to remain on the medical device subsequent to movement of the medical device out of the sealed receptacle and through the reducing template, wiping off excess of the coating material. The kit further includes instructions for use.

In accordance with a preferred non-limiting embodiment of this disclosure, An apparatus for storing and coating a medical device is provided, which includes: a sealed receptacle having a proximal end and a distal end, the receptacle containing a coating material; the medical device having an outer profile, the medical device disposed and sealed within the sealed receptacle and immersed in the coating material, the sealed receptacle configured to be unsealed or opened to enable the medical device to pass through at a time of use; and a reducing template having a cross-sectional inner profile, the reducing template adapted to receive the medical device and wipe excess of the coating material from the medical device; wherein an area defined by the cross-sectional inner profile of the reducing template is greater than an area defined by the outer profile of the medical device by a predetermined amount forming a gap area; wherein the predetermined amount forming the gap area is determined at least in part by a thickness of the coating material desired to remain on the medical device subsequent to movement of the medical device out of the sealed receptacle and through the reducing template, wiping off excess coating material; and wherein the sealed receptacle further comprises a catheter cap disposed within the sealed receptacle and sealing a lumen of the catheter.

In accordance with another preferred, non-limiting embodiment of this disclosure, a method of coating a medical device is provided, which includes the steps of: providing a storing and coating apparatus that comprises a sealed receptacle having a proximal end and a distal end, the receptacle containing a coating material; the medical device having an outer profile, the medical device disposed and sealed within the sealed receptacle and immersed in the coating material, the sealed receptacle configured to be unsealed or opened to enable the medical device to pass through at a time of use; and a reducing template having a cross-sectional inner profile, the reducing template adapted to receive the medical device and wipe excess of the coating material from the medical device; wherein an area defined by the cross-sectional inner profile of the reducing template is greater than an area defined by the outer profile of the medical device by a predetermined amount forming a gap area; and wherein the predetermined amount forming the gap area is determined at least in part by a thickness of the coating material desired to remain on the medical device subsequent to movement of the medical device out of the sealed receptacle and through the reducing template, wiping off excess of the coating material; and withdrawing the medical device through the reducing template causing the reducing template to regulate the thickness of the coating material formed on the medical device by wiping excess of the coating material from the medical device, wherein the method optionally further comprises the step of unsealing or opening the sealed receptacle prior to withdrawing the medical device through the reducing template.

In accordance with another preferred, non-limiting embodiment of this disclosure, a kit for coating a medical device is provided, wherein the kit includes: (a) a coating material; (b) the medical device; (c) a dispenser comprising a sealed receptacle containing the coating material and the medical device; a reducing template having a cross-sectional inner profile, the reducing template adapted to receive the medical device and wipe excess of the coating material from the medical device; wherein an area defined by the cross-sectional inner profile of the reducing template is greater than an area defined by the outer profile of the medical device by a predetermined amount forming a gap area; and wherein the predetermined amount forming the gap area is determined at least in part by a thickness of the coating material desired to remain on the medical device subsequent to movement of the medical device out of the sealed receptacle and through the reducing template, wiping off excess of the coating material; and (d) instructions for use.

### BRIEF DESCRIPTION OF THE DRAWINGS

The aforementioned features and advantages, and other features and aspects of the present invention, will become better understood with regard to the following description and accompanying drawings, wherein:
**FIG. 1** is a diagrammatic illustration of a medical device, according to one embodiment of the present invention;
**FIG. 2** is a cross-sectional view of the medical device in accordance with one aspect of the present invention;
**FIG. 3** is a cross-sectional view of the medical device in accordance with another aspect of the present invention;
**FIG. 4** is a flow chart illustrating a method of making a coated medical device, in accordance with one embodiment of the present invention;
**FIG. 5** is a flow chart illustrating a variation of the method of **FIG. 4** using an applicator in accordance with one embodiment of the present invention;
**FIG. 6A** is a diagrammatic illustration of an applicator in accordance with one embodiment of the present invention;
**FIG. 6B** is a diagrammatic illustration of an applicator in accordance with another embodiment of the present invention;
**FIG. 6C** is a diagrammatic illustration of an applicator in accordance with another embodiment of the present invention;
**FIG. 7** **is** a flow chart illustrating a variation of the method of **FIG. 4** using a cap stylet in accordance with one embodiment of the present invention;
**FIG. 8** is a diagrammatic illustration of a cap stylet in accordance with one embodiment of the present invention;
**FIG. 9** is a flow chart illustrating a method of applying a coating to a catheter using an applicator and cap stylet in accordance with one embodiment of the present invention;
**FIG. 10** is a diagrammatic illustration of the interaction of a catheter, applicator, and cap stylet in accordance with one embodiment of the present invention;
**FIG. 11** is a flow chart illustrating a variation of the method of **FIG. 4****,** in accordance with one embodiment of the present invention;
**FIG. 12** is a diagrammatic illustration of an apparatus that includes an outer container and an inert gas outer package, in accordance with one embodiment of the present invention;
**FIG. 13** is a flow chart illustrating another variation of the method of **FIG. 4****,** in accordance with one embodiment of the present invention;
**FIG. 14** is a cross sectional view of a coated medical device in accordance with one embodiment of the present invention;
**FIG. 15A** is a diagrammatic illustration of a side view of an apparatus for coating a medical device that includes a sealed receptacle with a reducing template and a crimp seal, in accordance with an embodiment of the present invention;
**FIG. 15B** is a diagrammatic illustration of a cross-sectional view of the apparatus depicted in **FIG 15A****;**
**FIG. 15C** is a diagrammatic illustration of a cross-sectional view of a distal end of the apparatus depicted in **FIG. 15A****;**
**FIG. 16A** is a diagrammatic illustration of the apparatus depicted in **FIGS. 15A-****15C** in use as the catheter shaft is being withdrawn from the apparatus and the medical device is within the reducing template;
**FIG. 16B** is a diagrammatic illustration of the apparatus depicted in **FIGS. 15A-****15C** after the catheter shaft has been completely withdrawn from the reducing template and the medical device is fully coated;
**FIG. 17** is a diagrammatic illustration of a cross-sectional view of a different apparatus for coating a medical device that includes a catheter cap and a stylet in one piece, in accordance with another embodiment of the present invention;
**FIG. 18** is a diagrammatic illustration of another apparatus for coating a medical device, wherein the distal end of the sealed receptacle and the catheter cap are formed of the receptacle wall, in accordance with another embodiment of the present invention;
**FIG. 19** is a diagrammatic illustration of another apparatus for coating a medical device, wherein the catheter cap and the stylet are in one piece with the end cap, in accordance with another embodiment of the present invention;
**FIG. 20** is a diagrammatic illustration of a cross-sectional view of another apparatus for coating a medical device, wherein there the end cap is substantially disposed within a receptacle wall, in accordance with another embodiment of the present invention;
**FIG. 21A** is a diagrammatic illustration of a distal end of the sealed receptacle wherein the proximal end is formed of the receptacle wall, according to aspects of the present invention;
**FIG. 21B** is a diagrammatic illustration of a distal end of the sealed receptacle wherein the proximal end is formed of an end cap fitting externally over the receptacle wall, according to aspects of the present invention;
**FIG. 21C** is a diagrammatic illustration of a distal end of the sealed receptacle wherein the distal end is formed of an end cap fitting internally within the receptacle wall, according to aspects of the present invention;
**FIG. 21D** is a diagrammatic illustration of a distal end of the sealed receptacle wherein the distal seal is a crimp seal, according to aspects of the present invention;
**FIG. 22A** is a diagrammatic illustration of a proximal end of the sealed receptacle, wherein the reducing template forms a portion of the sealed receptacle and a proximal seal forms the proximal end of the sealed receptacle, according to an aspect of the present invention;
**FIG. 22B** is a diagrammatic illustration of a proximal end of the sealed receptacle wherein the proximal seal is disposed within the reducing template and the apparatus includes a shrink tubing proximal end cover, according to aspects of the present invention;
**FIG. 22C** is a diagrammatic illustration of a proximal end of the sealed receptacle, wherein the proximal seal is a clamp seal disposed externally around the reducing template and the apparatus includes a proximal end cover, according to aspects of the present invention;
**FIG. 23A** is a diagrammatic illustration of a cross-sectional view along the reducing template when a stent is being withdrawn through the reducing template as shown from a different perspective in **FIG. 16A****;**
**FIG. 23B** is a diagrammatic illustration of the same cross-sectional view when the medical device is a balloon, according to aspects of the present invention;
**FIG. 24A** is a diagrammatic illustration of a side view of another apparatus for coating a medical device including a sleeve, a proximal seal and a seal breaching mechanism, according to aspects of the present invention;
**FIG. 24B** is an enlarged view of a portion of the apparatus depicted in **FIG. 24A****;**
**FIG. 25A** is a diagrammatic illustration of a proximal end of the apparatus depicted in **FIG. 24A** with the sleeve, the proximal seal and receptacle in an assembly configuration;
**FIG. 25B** is a diagrammatic illustration of a proximal end of the apparatus with the sleeve, the proximal seal and the sealed receptacle in a pre-use configuration for storage, shipping, etc.;
**FIG. 25C** is a diagrammatic illustration of a proximal end of the apparatus in an activation configuration after the apparatus has been activated;
**FIG. 26** is a diagrammatic illustration of some suitable shapes for a cross-sectional inner profile of a reducing template, according to aspects of the present invention;
**FIG. 27A** is a diagrammatic illustration of a side cross-sectional view of an apparatus for coating a surgical mesh in accordance with another embodiment of the present invention;
**FIG. 27B** is a diagrammatic illustration of a perspective view of the apparatus depicted in **FIG. 27A** after the device is activated and a proximal seal is breached;
**FIG. 27C** is an enlarged diagrammatic cross-sectional view along the reducing template of the apparatus depicted in **FIGS. 27A** and **27B****;** and
**FIG. 28** is a flow chart illustrating a method of coating a medical device using the apparatus depicted in **FIG. 15A****,** in accordance with one embodiment of the present invention.

### DETAILED DESCRIPTION

An illustrative embodiment of the present invention relates to the provision of a coating on an insertable or implantable medical device. An apparatus includes a sealed receptacle that contains and can preserve a coating material (if necessary) reducing the need for preservatives in the coating material. The apparatus forms a complete coating on the medical device shortly before insertion or implantation to reduce degradation of the coating and alleviate the need for preservative components in the applied coating. This is achieved by moving the medical device through a reducing template to regulate the amount coating material on the device as it is removed from the apparatus. The coating can include a bio-absorbable carrier component. In addition to the bio-absorbable carrier component, a therapeutic agent component can also be provided. However, the coating is not limited to a bio-absorbable carrier component or a therapeutic agent component. Rather, any variation of coating formed with application of a relatively liquid or fluent material that is desired for application to a medical device can be applied using the apparatus and method of the present invention. The coated medical device can be insertable or implantable in a patient to affect controlled delivery of the coating to the patient, or can be for external use.

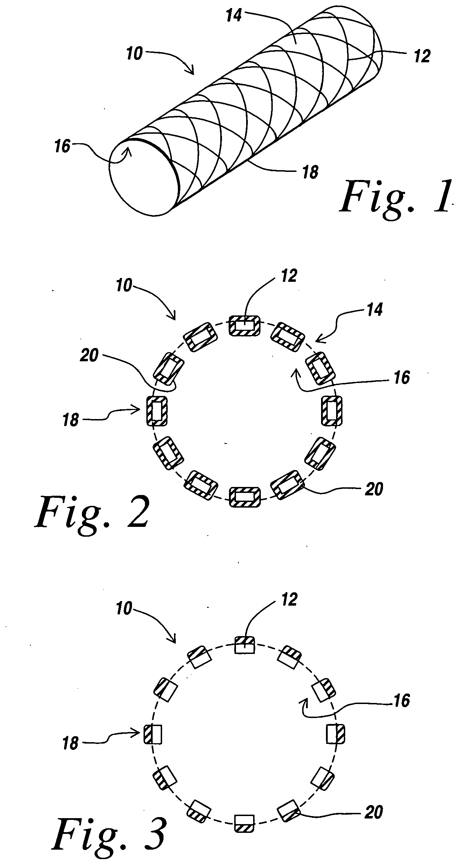

**FIGS. 1** through **28****,** wherein like parts are designated by like reference numerals throughout, illustrate example embodiments of apparatus and a corresponding method for coating a medical device, along with representative coated medical device examples. Although the present invention will be described with reference to the example embodiments illustrated in the figures, it should be understood that many alternative forms can embody the present invention. One of ordinary skill in the art will additionally appreciate different ways to alter the parameters of the embodiments disclosed, such as the size, shape, or type of elements or materials, in a manner still in keeping with the spirit and scope of the present invention.

**FIG. 1** illustrates a stent 10 in accordance with one aspect of the present invention. The stent 10 is representative of a medical device that is suitable for having a coating applied thereon to affect a beneficial result. The stent 10 is formed of a series of interconnected struts 12 having gaps 14 formed therebetween. The stent 10 is generally cylindrically shaped. Accordingly, the stent 10 maintains an interior surface 16 and an exterior surface 18.

One of ordinary skill in the art will appreciate that the illustrative stent 10 is merely exemplary of a number of different types of stents available in the industry. For example, the strut 12 structure can vary substantially. The material of the stent can also vary from a metal, such as stainless steel, Nitinol, nickel, tantalum, magnesium, and titanium alloys, to cobalt chromium alloy, ceramic, plastic, and polymer type materials. One of ordinary skill in the art will further appreciate that the present invention is not limited to use with stents. Instead, the present invention has application with a wide variety of medical devices. For purposes of clarity, the following description will refer to a stent as the exemplar medical device. The terms medical device and stent are interchangeable with regard to the applicability of the present invention. Accordingly, reference to one or another of the stent, or the medical device, is not intended to unduly limit the invention to the specific embodiment described. Furthermore, the term medical device is intended to apply to all medical devices that can be coated in the manner described herein, including but not limited to stents, balloons, grafts, sutures, catheters, surgical instruments, and the like. As such, the present invention is not limited to the particular medical devices utilized in the specific examples herein, other than to the extent required to make the specific embodiment operational.

**FIG. 2** illustrates one example embodiment of the stent 10 having a coating 20 applied thereon in accordance with an aspect of the present invention. **FIG. 3** is likewise an alternative embodiment of the stent 10 having the coating 20 also applied thereon. The coating 20 is applied to the medical device, such as the stent 10, to provide the stent 10 with different surface properties, and also to provide a vehicle for therapeutic applications.

In **FIG. 2****,** the coating 20 is applied on both the interior surface 16 and the exterior surface 18 of the strut 12 forming the stent 10. In other words, the coating 20 in **FIG. 2** substantially encapsulates the struts 12 of the stent 10. In **FIG. 3****,** the coating 20 is applied only on the exterior surface 18 of the stent 10, and not on the interior surface 16 of the stent 10. The coating 20 in both configurations is the same coating; the difference is merely the portion of the stent 10 that is covered by the coating 20. One of ordinary skill in the art will appreciate that the coating 20 as described throughout this description can be applied in both manners shown in **FIG. 2** and **FIG. 3****,** in addition to other configurations such as, partially covering select portions of the stent 10 structure. All such configurations are described by the coating 20 reference.

In some instances of the resulting coated medical device, the stent 10 includes the coating 20, which is bio-absorbable. The coating 20 has a bio-absorbable carrier component, and can also include a therapeutic agent component that can also be bio-absorbable. When applied to a medical device such as a stent 10, it is often desirable for the coating to inhibit or prevent restenosis. Restenosis is a condition whereby the blood vessel experiences undesirable cellular remodeling after injury. When a stent is implanted in a blood vessel, and expanded, the stent itself may cause some injury to the blood vessel. The treated vessel typically has a lesion present which can contribute to the inflammation and extent of cellular remodeling. The end result is that the tissue has an inflammatory response to the conditions. Thus, when a stent is implanted, there is often a need for the stent to include a coating that inhibits inflammation, or is non-inflammatory, and prevents restenosis. These coatings have been provided using a number of different approaches as previously described in the Background. However, none of the prior coatings have utilized a bio-absorbable carrier component to create a bio-absorbable coating with suitable non-inflammatory properties for controlled release of a therapeutic agent, and in a manner consistent with the present invention.

The coating can also include a therapeutic agent component. The therapeutic agent component mixes with the bio-absorbable carrier component as described later herein. The therapeutic agent component can take a number of different forms including but not limited to anti-oxidants, anti-inflammatory agents, anti-coagulant agents, drugs to alter lipid metabolism, anti-proliferatives, anti-neoplastics, tissue growth stimulants, functional protein/factor delivery agents, anti-infective agents, imaging agents, anesthetic agents, therapeutic agents, tissue absorption enhancers, anti-adhesion agents, germicides, antiseptics, proteoglycans, GAG's, gene delivery (polynucleotides), polysaccharides (e.g., heparin), anti-migratory agents, pro-healing agents, ECM/protein production inhibitors, analgesics, prodrugs, and any additional desired therapeutic agents such as those listed in Table 1 below.

**Table #1**

| **CLASS** | **EXAMPLES** |
|---|---|
| Antioxidants | Alpha-tocopherol, lazaroid, probucol, phenolic antioxidant, resveretrol, AGI-1067, vitamin E |
| Antihypertensive Agents | Diltiazem, nifedipine, verapamil |
| Antiinflammatory Agents | Glucocorticoids (e.g. dexamethazone, methylprednisolone), leflunomide, NSAIDS, ibuprofen, acetaminophen, hydrocortizone acetate, hydrocortizone sodium phosphate, macrophage-targeted bisphosphonates, cyclosporine, vocolosporine |
| Growth Factor Antagonists | Angiopeptin, trapidil, suramin |
| Antiplatelet Agents | Aspirin, dipyridamole, ticlopidine, clopidogrel, GP IIb/IIIa inhibitors, abcximab |
| Anticoagulant Agents | Bivalirudin, heparin (low molecular weight and unfractionated), wafarin, hirudin, enoxaparin, citrate |
| Thrombolytic Agents | Alteplase, reteplase, streptase, urokinase, TPA, citrate |
| Drugs to Alter Lipid Metabolism (e.g. statins) | Fluvastatin, colestipol, lovastatin, atorvastatin, amlopidine |
| ACE Inhibitors | Elanapril, fosinopril, cilazapril |
| Antihypertensive Agents | Prazosin, doxazosin |
| Antiproliferatives and Antineoplastics | Cyclosporine, cochicine, mitomycin C, sirolimus micophenonolic acid, rapamycin, everolimus, tacrolimus, paclitaxel, QP-2, actinomycin, estradiols, dexamethasone, methatrexate, cilostazol, prednisone, cyclosporine, doxorubicin, ranpirnas, troglitzon, valsarten, pemirolast, C-MYC antisense, angiopeptin, vincristine, PCNA ribozyme, 2-chloro-deoxyadenosine, vocolosporine |
| Tissue growth stimulants | Bone morphogeneic protein, fibroblast growth factor |
| Promotion of hollow organ occlusion or thrombosis | Alcohol, surgical sealant polymers, polyvinyl particles, 2-octyl cyanoacrylate, hydrogels, collagen, liposomes |
| Functional Protein/Factor delivery | Insulin, human growth hormone, estradiols, nitric oxide, endothelial progenitor cell antibodies |
| Second messenger targeting | Protein kinase inhibitors |
| Angiogenic | Angiopoetin, VEGF |
| Anti-Angiogenic | Endostatin |
| Inhibitation of Protein Synthesis/ECM formation | Halofuginone, prolyl hydroxylase inhibitors, C-proteinase inhibitors |
| Antiinfective Agents | Penicillin, gentamycin, adriamycin, cefazolin, amikacin, ceftazidime, tobramycin, levofloxacin, silver, copper, hydroxyapatite, vancomycin, ciprofloxacin, rifampin, mupirocin, RIP, kanamycin, brominated furonone, algae byproducts, bacitracin, oxacillin, nafcillin, floxacillin, clindamycin, cephradin, neomycin, methicillin, oxytetracycline hydrochloride, Selenium. |
| Gene Delivery | Genes for nitric oxide synthase, human growth hormone, antisense oligonucleotides |
| Local Tissue perfusion | Alcohol, H2O, saline, fish oils, vegetable oils, liposomes |
| Nitric oxide Donor | NCX 4016 - nitric oxide donor derivative of aspirin, |
| Derivatives | SNAP |
| Gases | Nitric oxide, compound solutions |
| Imaging Agents | Halogenated xanthenes, diatrizoate meglumine, diatrizoate sodium |
| Anesthetic Agents | Lidocaine, benzocaine |
| Descaling Agents | Nitric acid, acetic acid, hypochlorite |
| Anti-Fibrotic Agents | Interferon gamma -1b, Interluekin - 10 |
| Immunosuppressive/Immu nomodulatory Agents | Cyclosporine, rapamycin, mycophenolate motefil, leflunomide, tacrolimus, tranilast, interferon gamma-1b, mizoribine, vocolosporine |
| Chemotherapeutic Agents | Doxorubicin, paclitaxel, tacrolimus, sirolimus, fludarabine, ranpirnase |
| Tissue Absorption Enhancers | Fish oil, squid oil, omega-3 fatty acids, vegetable oils, lipophilic and hydrophilic solutions suitable for enhancing medication tissue absorption, distribution and permeation |
| Anti-Adhesion Agents | Hyaluronic acid, human plasma derived surgical sealants, and agents comprised of hyaluronate and carboxymethylcellulose that are combined with dimethylaminopropyl, ehtylcarbodimide, hydrochloride, PLA, PLGA |
| Ribonucleases | Ranpirnase |
| Germicides | Betadine, iodine, sliver nitrate, furan derivatives, nitrofurazone, benzalkonium chloride, benzoic acid, salicylic acid, hypochlorites, peroxides, thiosulfates, salicylanilide |
| Antiseptics | Selenium |
| Analgesics | Bupivicaine, naproxen, ibuprofen, acetylsalicylic acid |

Some specific examples of therapeutic agents useful in the anti-restenosis realm include cerivastatin, cilostazol, fluvastatin, lovastatin, paclitaxel, pravastatin, rapamycin, a rapamycin carbohydrate derivative, a rapamycin derivative, everolimus, seco-rapamycin, seco-everolimus, and simvastatin, as well as derivatives and prodrugs of any of these examples and any of the above noted agents.

It should also be noted that the present description makes use of the stent 10 as an example of a medical device that can be coated with the coating 20 of the present invention. However, the present invention is not limited to use with the stent 10. Instead, any number of other insertable or implantable medical devices can be coated in accordance with the teachings of the present invention with the described coating 20. Such medical devices include needles, stylets, catheters, grafts, balloons, prostheses, stents, other medical device implants, and the like. Implantation refers to both temporarily implantable medical devices, as well as permanently implantable medical devices. Insertion refers to medical devices that are place within a living system. In the instance of the example stent 10, a common requirement of stents is that they include some substance or agent that inhibits restenosis. Accordingly, the example coating 20 as described is directed toward the reduction or the elimination of restenosis. However, one of ordinary skill in the art will appreciate that the coating 20 can have other therapeutic or biological benefits. For example, the coating 20 can alternately be used as a lubricant that eases the insertion of a device or minimizes irritation caused by a device. The composition of the coating 20 is simply modified or mixed in a different manner to result in a different biological or physical effect.

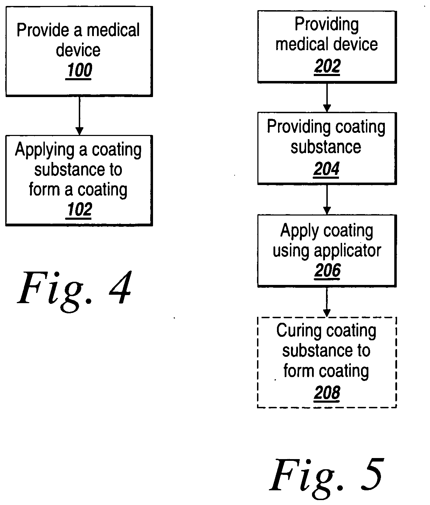

**FIG. 4** illustrates one method of making a coated medical device, in the form of the coated stent 10. The process involves providing a medical device, such as the stent 10 (step 100). A coating, such as coating 20, is then applied to the medical device (step 102). One of ordinary skill in the art will appreciate that this basic method of application of a coating to a medical device such as the stent 10 can have a number of different variations falling within the process described. Depending on the particular application, the stent 10 with the coating 20 applied thereon can be implanted after the coating 20 is applied, or additional steps such as curing, sterilization, and removal of solvent can be applied to further prepare the stent 10 and coating 20. Furthermore, if the coating 20 includes a therapeutic agent that requires some form of activation (such as UV light), such actions can be implemented accordingly.

In one embodiment of the present invention, applying the coating to the medical device involves using an applicator to apply the coating. The use of an applicator allows for application of a coating having improved uniformity and coverage. An exemplary method of this can be seen in **FIG. 5****.** The method involves providing a medical device onto which a coating is to be applied (step 202); providing a coating substance for application onto the medical device (step 204); and applying the coating substance to the medical device using an applicator (step 206). In certain embodiments, the method may further include the step of curing the coating substance to form a coating on the medical device (step 208).

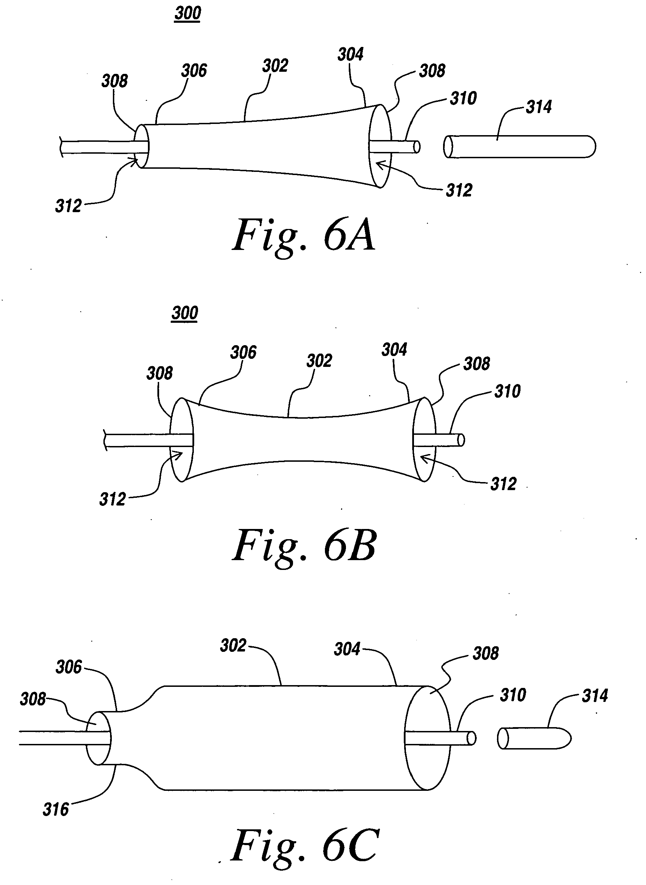

An exemplary embodiment of an applicator 300 can be seen in **FIG. 6A****.** The applicator 300 is formed of a sheath 302 having a first end 304, a second end 306 and a lumen 308 between the first 304 and second 306 ends. The first end 304 is flared and has a cross-sectional area greater than a cross sectional area of a portion of the lumen 308. The sheath 302 is sized and dimensioned to fit over the medical device 310, while providing a clearance 312 between the sheath 302 and the medical device 310 for receiving a coating substance for application to the medical device 310. In some embodiments, the second end 306 may also be flared and have a cross-sectional area greater than a cross-sectional area of at least a portion of the lumen 308, as can be seen in **FIG. 6B****.** In some embodiments, the second end 306 may also be necked down and have a cross-sectional area less than a cross-sectional area of at least a portion of the lumen 308, as can be seen in **FIG. 6C****.** The length of the land 316 on the necked down section of the second end 306 of the applicator 300 can be sized to deposit a consistent coating weight. The first end 304 can optionally be flared to ease the passage over the device 310. Examples of medical devices 310 on which the applicator 300 may be used include stents and catheters. In certain embodiments, a coating is applied to a stent that has been positioned on the end of a catheter. Preferably, the applicator 300 is formed of plastic but other suitable material that can be formed into the desired configuration can be used. This particular coating method can be used for any device which is substantially cylindrical in geometry, such as devices like guide wires, stylets, as well as grafts, fibers, and the like.

In the present embodiment the cross-sectional shape of the applicator is circular giving the applicator a funnel or trumpet like shape. Other suitable cross sectional shapes include polygonal shapes such as hexagonal, octagonal, or the like, expandable cross sections that contact the device or change dimensions as they pass over the device, and/or substantially irregular shapes such as fingers or bristles that wipe off excess coating. Other possible shapes and configurations will be apparent to one skilled in the art given the benefit of this disclosure.

In use, the coating is applied by placing the applicator 300 onto the medical device 310 and then filling the applicator 300 with the coating substance. The flared nature of the first end 304 assists in providing a larger opening for receiving the coating substance and directing it onto the medical device. The coating substance may be placed into the applicator 300, for example, at flared first end 304, or be placed onto the medical device 310 directly. In certain embodiments the coating substance is delivered using a metering device, such as a dispenser, so that the amount of coating, and in certain cases, dosage of a therapeutic agent, can be controlled. In other embodiments, the design, dimensions and material properties of the applicator can be used to control the dosage of a therapeutic agent.

In the present embodiment the applicator 300 is configured to slide onto or over the medical device 310. In other embodiments, the applicator 300 may be formed of two halves that are joined together around the medical device 310. Other possible configurations will be apparent to one skilled in the art given the benefit of this disclosure.

In certain embodiments, after the applicator 300 has been filled with coating substance, the applicator 300 can be removed. In the present embodiment, wherein the applicator 300 is configured to slide onto the medical device 310, removing the applicator 300 is performed by sliding the applicator 300 off the medical device 310. Alternately, the coating substance may be applied directly to the medical device 310 and the applicator 300 is then slid over the medical device 310 to spread the coating substance over the medical device 310. In this embodiment, the clearance between the sheath 302 and the medical device 310 is dimensioned and sized to leave a residual coating of the coating substance on the medical device 310 as the applicator 300 is slid over the medical device 310. Preferably, the clearance is between 0.0001 to 0.1 inches. More preferably, the clearance is between 0.001 to 0.01 inches. In certain embodiments the uniformity and coverage of such a residual coating can be improved by sliding the applicator 300 over the medical device 310 with a twisting motion.

In certain embodiments, as set forth in step 208 of **FIG. 5****,** once the coating substance has been applied to the medical device, the coating substance is cured or activated to form the coating on the medical device. Curing or activating can be performed after the applicator has been removed, or with the applicator still in place over the medical device. Curing or activating with respect to the present invention generally refers to thickening, hardening, or drying of a material brought about by heat, UV, reactive gases, exposure to air, or chemical means.

In some embodiments, once the coating has been formed on the medical device 310, a protective sleeve 314 is placed over the medical device 310 to protect the coating on the medical device 310 during further handling. In an exemplary embodiment, the protective sleeve 314 is formed of plastic, and sized and dimensioned to fit over the medical device 310. Other suitable implementations will be apparent to one skilled in the art given the benefit of this disclosure.

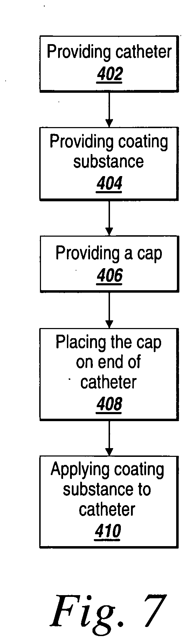

In certain embodiments wherein a coating is being applied to a catheter, a cap, such as a coating cap or cap stylet, may be used when applying the coating substance. When placed on the end of a catheter, the cap prevents coating substance from penetrating the lumen at the end of the catheter. A cap stylet can be a section of tubing diametrically designed to fit over the end of the catheter and long enough to prevent coating material from flowing into the catheter lumen. An exemplary embodiment of such a method can be seen in **FIG. 7****.** In this embodiment, the method involves providing a catheter onto which a coating is to be applied (step 402), providing a coating substance for application onto the catheter (step 404), providing a cap configured to fit onto an end of the catheter (step 406); placing the cap onto an end of the catheter (step 408), and applying the coating substance onto the catheter (step 410).

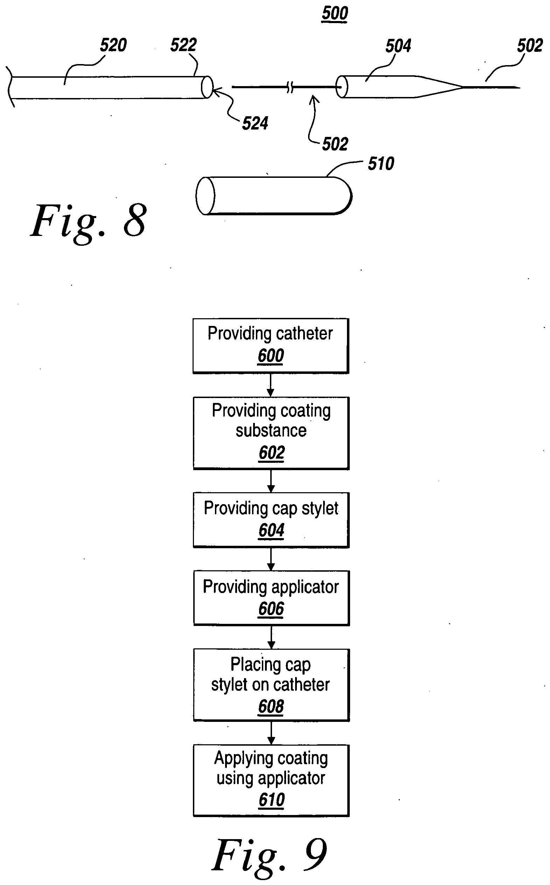

The cap comprises a section of tubing configured to fit on the end of the catheter to seal the lumen at the end of the catheter during the application of a coating. The cap can optionally be attached to a stylet, as in the case with a cap stylet or the cap can be separate from the stylet. The cap can optionally be closed on one end. An exemplary embodiment of a cap and its interaction with a catheter can be seen in **FIG. 8****.** In this embodiment the cap is a cap stylet 500. The catheter 520 has a proximal end (not shown), a distal end 522, and a lumen 524 between the proximal and distal ends. The cap stylet 500 features a stylet 502 configured to fill the lumen 524 of the catheter 520; and a section of tubing 504 attached to the stylet 502 sized and dimensioned to be fitted on the end of the catheter 520 to seal the lumen 524 of the catheter 520. In certain embodiments, the section of tubing 504 is sized to pinch fit on the end of a catheter 520. Alternately, the cap can snap or interference fit on the end of the catheter. When placed on the end of a catheter 520, the cap stylet 500 prevents the coating substance from wicking into the lumen 524 at the end of the catheter 520 as a coating is applied. If the coating substance gets into the lumen 524 it could create an obstruction that may adversely effect the operation of the catheter 520. Preferably, the cap stylet 500 is placed on the distal end 522 of the catheter 520, which is to be inserted into a patient, and is thus coated. In certain embodiments wherein the whole catheter is to be coated, a cap stylet 500 can be placed on each end of the catheter 520.

Once the cap, in this case a cap stylet 500, has been placed on the end of the catheter 520, the coating can then be applied to the catheter (step 410 of **FIG. 7****).** In certain embodiments this involves using an applicator as set forth above. The coating may also be applied by dip coating (including submersing, surrounding, bathing), spray coating, printing, wiping, electrostatic coating, brushing, painting, pipetting, or any means suitable for applying the coating substance.

Once the coating substance has been applied, the coating substance can then be cured as discussed above. Likewise, in some embodiments a protective sleeve 510 may be placed on the catheter 520 to protect the coating.

Another exemplary embodiment of a method, wherein an applicator and a cap stylet are used in forming a coating on a catheter, can bee seen in **FIG. 9****.** In this embodiment, the method includes providing a catheter (step 600), providing the coating substance for application onto the catheter (step 602), providing a cap stylet configured to fit onto an end of the catheter (step 604), providing an applicator configured to apply a coating to the catheter (step 606), placing the cap stylet onto an end of the catheter (step 608) and applying the coating substance onto the catheter using the applicator (step 610).

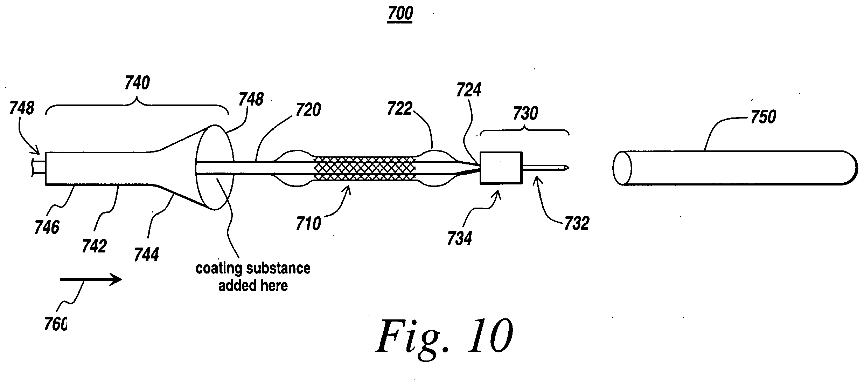

The methodology of **FIG. 9** may be better understood if viewed in conjunction with the exemplary embodiment of **FIG. 10** of a system 700 for applying a coating to a medical device. In this instance, a catheter 720 has a stent 710 pre-positioned on the distal end 722 of the catheter for implantation in a patient. A cap stylet 730 is placed onto the distal end 722 of the catheter 720. The cap stylet 730 features a stylet 732 configured to fill a lumen 724 of the catheter 720, and a section of tubing 734 optionally attached to the stylet 732 sized and dimensioned to be fitted on an end of the catheter 720 to seal the lumen 724 of the catheter 720. An applicator 740 is then slid onto the catheter 720 beyond the stent 710. The applicator 740 features a sheath 742 having a first end 744, a second end 746 and a lumen 748 between the first 744 and second 746 ends. The first end 744 is optionally flared and has a cross-sectional area greater than a cross-sectional area of at least a portion of the lumen 748. The second end 746 is optionally necked down to a dimension less than the cross sectional area of at least a portion of the lumen 748. The sheath 742 is sized and dimensioned to fit over the catheter 720, providing a clearance between the sheath 742 and the catheter 720 for receiving a coating substance for application to the catheter. The coating substance is then applied. In this embodiment, the applicator 740 is filled with coating substance at the flared first end 744 using a metering device, such as a dispenser, to ensure the proper amount of coating substance is applied. Alternately, the coating substance may be applied directly to the catheter 720 or the stent 710. The applicator 740 is then slid off the catheter 720 over the stent 710 while optionally removing the cap stylet 730 in the direction of arrow 760 using, for example, a twisting motion. The clearance between the sheath 742 and the catheter 720 is sized and dimensioned to leave a residual coating of the coating substance as the applicator 740 is slid over the catheter 720. In another embodiment, the clearance between the optionally necked down second end 746 of the sheath 742 and the catheter 720 is sized and dimensioned to leave a residual coating of the coating substance as the applicator 740 is slid over the catheter 720.

In certain embodiments, once the coating substance has been applied, the coating substance may be cured as discussed above. Likewise, a protective sleeve 750 can be placed over the catheter 720 and stent 710 to protect the coating during further handling.

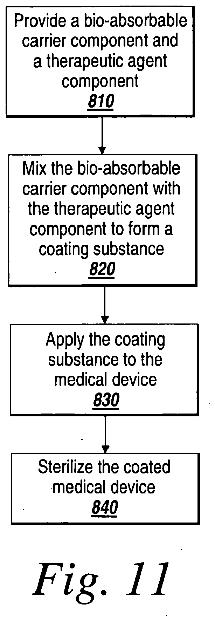

**FIG. 11** is a flowchart illustrating another example implementation of the method of **FIG. 4****.** In accordance with the steps illustrated in **FIG. 11****,** a bio-absorbable carrier component is provided along with a therapeutic agent component (step 810). The provision of the bio-absorbable carrier component and the provision of the therapeutic agent component can occur individually, or in combination, and can occur in any order or simultaneously. The bio-absorbable carrier component is mixed with the therapeutic agent component (or vice versa) to form a coating substance (step 820). The coating substance is applied to the medical device, such as a stent or catheter, to form the coating (step 830). The coated medical device is then sterilized using any number of different sterilization processes (step 840). For example, sterilization can be implemented utilizing ethylene oxide, gamma radiation, E beam, steam, gas plasma, vaporized hydrogen peroxide, or other physical, chemical, or mechanical means which results in the destruction or elimination of living microorganisms. One of ordinary skill in the art will appreciate that other sterilization processes can also be applied, and that those listed herein are merely examples of sterilization processes that result in a sterilization of the coated stent, preferably without having a detrimental effect on the coating 20. Furthermore, one of ordinary skill in the art will appreciate that the coating and device can be sterilized prior to application of the coating to the medical device.

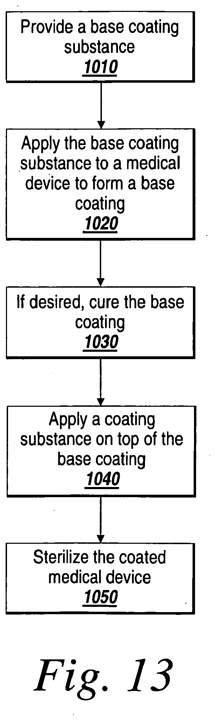

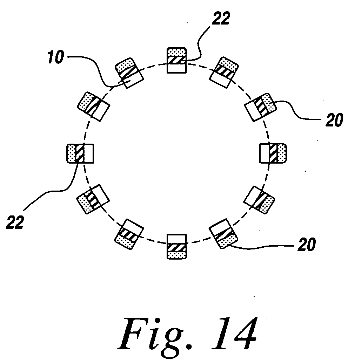

In accordance with another technique, a surface preparation or pre-treatment 22, as shown in **FIG. 14****,** is provided on a stent 10. More specifically and in reference to the flowchart of **FIG. 13****,** a pre-treatment substance or base coating is first provided (step 1010). The pre-treatment substance or base coating is applied to a medical device, such as the stent 10, to prepare the medical device surface for application of the coating (step 1020). If desired, the base coating or pre-treatment 22 is cured (step 1030). Curing methods can include processes such as application of UV light, heat, reactive gases, air or chemical means to cure the pre-treatment 22. A coating substance is then applied on top of the pre-treatment 22 (step 1040). The coated medical device is then sterilized using any number of sterilization processes as previously mentioned (step 1050).

**FIG. 14** illustrates the stent 10 having two coatings, specifically, the pre-treatment 22 and the coating 20. The pre-treatment 22 serves as a base or primer for the coating 20. The coating 20 conforms and adheres better to the pre-treatment 22 that conforms and adheres directly to the stent 10, especially if the coating 20 is not heat or UV cured. The pre-treatment can be formed of a number of different materials or substances. In accordance with one example embodiment of the present invention, the pre-treatment is formed of a bio-absorbable substance, such as a naturally occurring oil (e.g., fish oil). The bio-absorbable nature of the pre-treatment 22 results in the pre-treatment 22 ultimately being absorbed by the cells of the body tissue after the coating 20 has been absorbed.

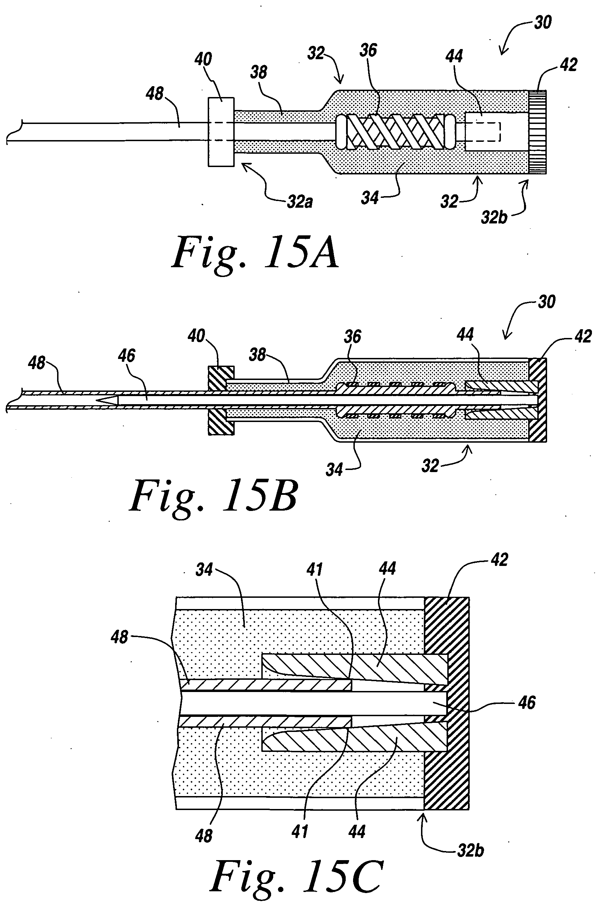

**FIGS. 15A** to **27C** illustrate additional embodiments of the present invention that each include an apparatus with a sealed receptacle holding a coating material and a medical device, and an applicator or reducing template. The medical device is submerged in the coating material, held in the sealed receptacle. Because the medical device is submerged in the coating material, the outer surfaces of the medical device are fully wetted by the coating material. The seal is broken or removed and the medical device is withdrawn through the reducing template. When the medical device is withdrawn through the reducing template, excess coating material is removed from the medical device, leaving a substantially uniform and complete coating on the medical device. By excess what is meant is an amount of coating material beyond that which is required or needed to attain the desired amount of coating material on the medical device after the device has been withdrawn from the apparatus of the present invention, or during a particular coating step of the coating process.

It should be noted that the sealed receptacle, as referenced throughout the present description, includes both receptacles with actual seals, as well as fully enclosed structures that are substantially impervious to the surrounding environment in terms of preserving or storing the coating material therein. As such, when the present description refers to unsealing, breaking the seal, removing the seal, or the like, such references include any method of penetrating the wall of the receptacle so as to allow the coating material to come into contact with the environment surrounding the receptacle. One of ordinary skill in the art will appreciate that the present invention is not limited to requiring an actual seal placed on to a receptacle. While this embodiment is included in the embodiments of the present invention, other equivalents will be apparent to those of ordinary skill in the art, and are intended to be anticipated by the present invention.

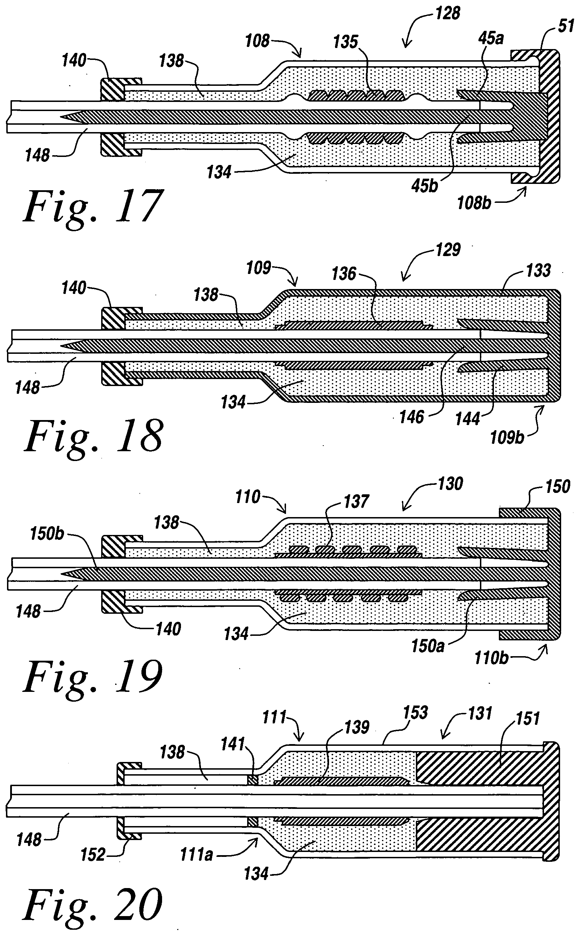

Furthermore, as discussed above, one of ordinary skill in the art will appreciate that illustrative stents depicted in **FIGS. 15A-15C****,** **16A****,** **16B****,** **17****,** **19****,** **23A****,** **24A, 24A** and **24B** are merely exemplary of a number of different types of stents available in the industry. For example, a strut structure can vary substantially. The material of the stent can also vary from a metal, such as stainless steel, Nitinol, nickel, tantalum, magnesium, and titanium alloys, to cobalt chromium alloy, ceramic, plastic, and polymer type materials. Additionally, one of ordinary skill in the art will further appreciate that illustrative balloons shown in **FIGS. 18, 19, 20****,** and **23B** are merely exemplary of a number of different types of balloons available in the industry that may be incorporated into exemplary embodiments of the present invention. Further, the present invention is not limited to stents and balloons, but may employ a wide variety of insertable or implantable medical devices including needles, catheters, grafts, meshes, various types of balloons, prostheses, various types of stents, and other medical devices, such as grafts, fibers, dialysis needles, surgical instruments, and the like. Implantation refers to both temporarily implantable medical devices, as well as permanently implantable medical devices. Insertion refers to medical devices that are placed within a living system. For purposes of clarity, the description of **FIGS. 15A-27C** will refer to a stent, a balloon a stent/balloon combination, and a surgical mesh as exemplar medical devices; however, reference to a particular medical device is not intended to unduly limit the invention to the specific embodiment described.

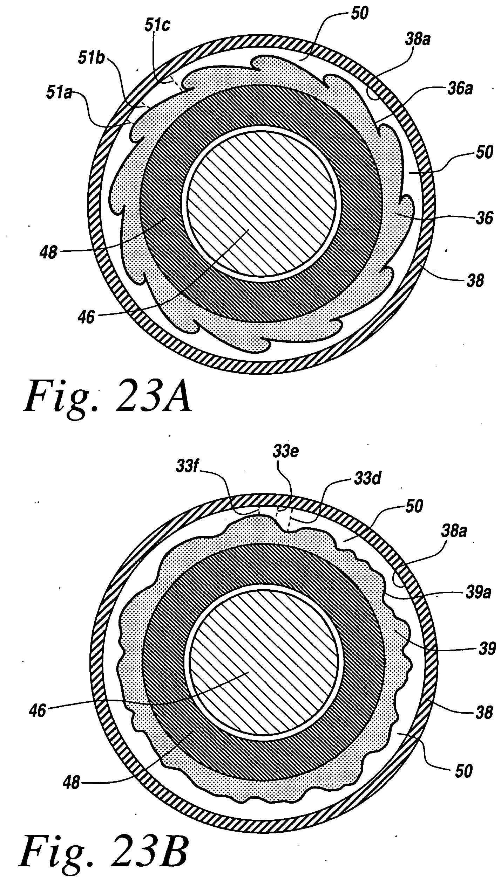

**FIGS. 15A, 15B** and **15C** diagrammatically illustrate an apparatus 30 for coating a medical device 36 with a coating material 34 in accordance with an embodiment of the present invention. **FIG. 15A** illustrates a side view of the apparatus 30, **FIG. 15B** illustrates a cross-sectional view of the apparatus 30, and **FIG. 15C** illustrates an enlarged cross-sectional view of a distal end of the apparatus. The apparatus 30 includes a sealed receptacle 32, a medical device 36 in the form of a stent and a reducing template 38. The sealed receptacle 32 has a proximal end 32a and a distal end 32b. The receptacle contains and preserves the coating material 34. The medical device has an outer profile 36a (see also **FIG. 23A****).** The medical device is disposed within the sealed receptacle 32 and immersed in the coating material 34. The reducing template 38 has a cross-sectional inner profile 38a (see also **FIG. 23A****).** The reducing template 38 is adapted to wipe excess coating material 34 from the medical device 36. An area defined by the cross-sectional inner profile 38a of the reducing template is greater than an area defined by the outer profile 36a of the medical device 36 by a predetermined amount forming a gap area 50 (see also **FIG. 23A****).** The predetermined amount forming the gap area 50 is determined at least in part by a thickness of coating material 34 desired to remain on the medical device subsequent to movement of the medical device 36 though the reducing template 38 and out of the sealed receptacle 32, wiping off excess coating material.

The coating material 34 may be susceptible to oxidation and/or other degradation that that can occur due to contact with air. The sealed receptacle 32 is sealed to prevent (or substantially hinder) air from entering the sealed receptacle 32 and contacting the coating material 34. The sealed receptacle 32 preserves the coating material 34, and contains the coating material 34 before use. The sealed receptacle 32 may be evacuated or filled with an inert gas prior to being sealed to ensure that no air interacts with the coating material 34 while it is held within the sealed receptacle 32. The sealed receptacle 32 may be formed of any material or any combination of materials that is substantially non-reactive with the coating material 34, that is at least substantially impermeable to oxygen, and that would contain and preserve the coating material 34. For example, the sealed receptacle 32 could be formed of glass, stainless steel, mixtures of polypropylene and polyvinyl alcohol (PP/PVA), Nylon, Pebax, polyolefins, rubbers, elastomers, fluoropolymers, etc.

As described above, the coating material 34 may be composed of any number of the bio-absorbable oils discussed previously. The coating material 34 may also include any number of the therapeutic agents discussed above and appearing in **Table #1,** as well as their analogs, derivatives, and prodrugs. Other possible coating materials including other oils, or non-oils, and other therapeutic agents not explicitly mentioned in this disclosure will be apparent to one skilled in the art given the benefit of this disclosure.

As illustrated by this embodiment, the medical device 36 may be mounted on a catheter and catheter shaft 48. The catheter shaft 48 facilitates handling of the medical device. In this embodiment the catheter shaft 48 forms a portion of the sealed receptacle 32.

The reducing template 38 may form a portion of the sealed receptacle or may be external to the sealed receptacle. In this embodiment, the reducing template 38 forms a portion of the proximal end 32a of the sealed receptacle 32. A cross-sectional inner profile 38a of the reducing template is chosen such that an area defined by the cross-sectional inner profile 38a of the reducing template is greater than an area defined by the outer profile 36a of the medical device by a predetermined amount forming a gap area 50. An explanation of the significance and function of the gap area 50 is presented below in the discussion of **FIGS. 23A** and **23B****.**

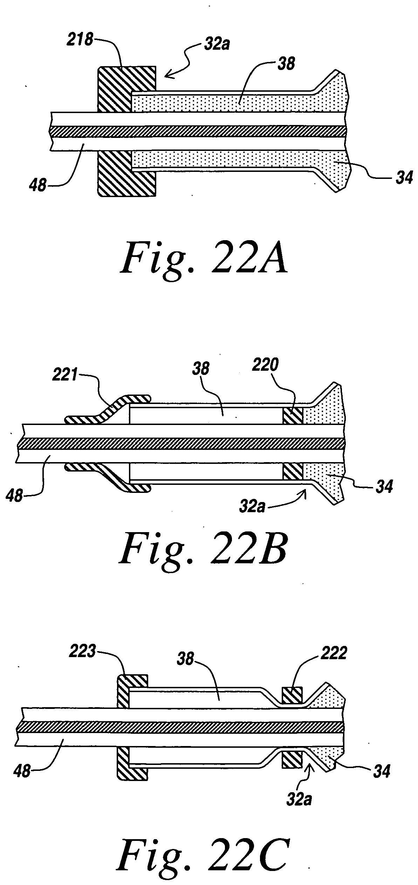

The sealed receptacle 32 may include one or more seals. In this embodiment, the sealed receptacle 32 includes a proximal seal or first seal 40 disposed at a proximal end 32a of the sealed receptacle. The first seal 40 seals and contains the coating material 34 until use. The first seal 40 may be constructed using any suitable material and using any suitable design that would prevent oxidation of the coating material 34 by preventing oxygen from entering the sealed receptacle 32, and that would contain the coating material 34 by preventing migration and leakage. Additionally, during the coating process, access to the reducing template 38 is required. The first seal 40 must be removable or alterable enabling access to the reducing template 38 during the coating process. In this embodiment, the first seal 40 is in contact with both the reducing template 38 and the catheter shaft 48 forming the proximal end 32a of the sealed receptacle. Other examples of designs and materials for a suitable first seal are presented below in the discussion of **FIGS. 22A-22D.** In addition, it should be noted that the first seal 40 can be a clamshell seal, a Thouy-Borst seal, a peelable seal, a compression seal, an extension seal, and/or a spring loaded seal.

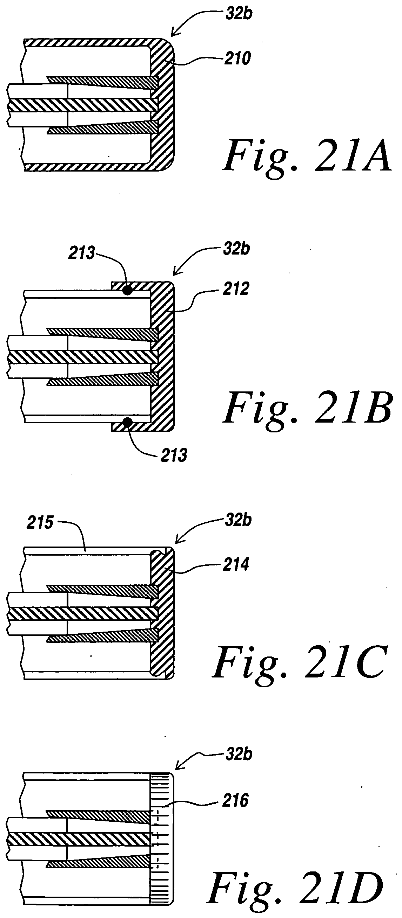

In the embodiment depicted in **FIGS. 15A, 15B** and **15C****,** the distal end 32b of the sealed receptacle 32 is substantially formed by a distal seal or second seal in the form of a crimp seal 42. In this embodiment the crimp seal 42 seals the distal end 32b of the sealed receptacle 32 and attaches a catheter cap 44 and a stylet 46 to the distal end 32b of the sealed receptacle. Although, the second seal is shown as a crimp seal 42, the second seal may be a compression-crimped fitting, may be a heat seal, may be made using fusing, shrink tubing, adhesives, ultrasonic and heat staking or may use any other suitable methods or materials for making a seal that are known in the art. The second seal prevents coating material 34 from leaking out from the apparatus 30 and prevents air from entering the sealed receptacle 32. Other examples of suitable embodiments for the distal end of the sealed receptacle are presented below in the discussion of **FIGS. 21A-21D****.**

**FIG. 15C** diagrammatically illustrates a cross-sectional view of the distal end 32b of the sealed receptacle depicted in **FIGS. 15A** and **15B****.** Both the stylet 46 and the catheter cap 44 may be fixed to the distal end 32b of the sealed receptacle. In this embodiment, both the stylet 46 and the catheter cap 44 are fixed to the distal end 32b of the sealed receptacle 32 using the crimp seal 42. A diameter of the stylet 46 is sized to fit within a lumen of the catheter shaft 48. The stylet 46 ensures that the catheter shaft 48 and the attached medical device 36 maintain a correct position within the apparatus 30 before use and the stylet 46 guides the motion of the catheter shaft 48 when the catheter shaft and the attached medical device 36 are withdrawn from the rest of the apparatus 30 through the reducing template 38. There may be an annular gap between the exterior of the stylet 46 and the interior lumen of the catheter shaft 48. If this annular gap is not sealed then the coating material 34 may be exposed to oxygen, degrading it, and the coating material 34 may seep into the annular gap, affecting how the catheter shaft 48 slides on the stylet 46. Seepage of the coating material 34 into this annular gap can result in the catheter shaft 48 becoming stuck on the stylet 46 and/or the catheter shaft 48 being damaged when it is slid off of the stylet 46. In this embodiment, the annular gap is sealed using a catheter cap 44. As shown in **FIG. 15C** a catheter cap 44 may be used to seal the gap between the catheter shaft 48 and the stylet 46. The catheter cap 44 is designed to prevent the coating material 34 from entering an interior lumen of the catheter shaft 48. An inner diameter of catheter cap 44 may be tapered such that it becomes smaller than an outer diameter of the catheter shaft 48 creating a friction fit seal 41. The catheter cap 44 may be made of Pebax, PET, PTFE, nylon, polyolefins or any other inert material known in the art that would form a suitable seal with a tip or end of the catheter shaft 48.

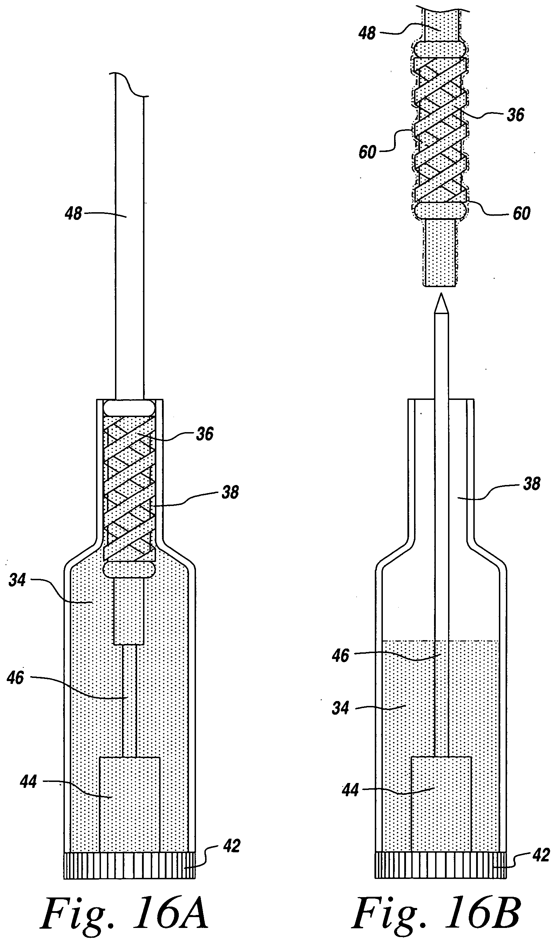

**FIGS. 16A** and **16B** diagrammatically illustrate a side view of the apparatus 30, depicted in **FIGS. 15A -15C****,** in use. In **FIG. 16A****,** the first seal 40 (not shown) has already been removed and the medical device 36 is being withdrawn through the reducing template 38. As the medical device 36 is withdrawn through the reducing template 38, excess coating material 34 is removed from the medical device 36. As the medical device 36 is withdrawn, a thickness of the coating material to remain on the device is partially determined by the gap area 50, as presented below in the discussion of **FIGS. 23A** and **23B****.** In **FIG. 16B** the medical device 36 and the catheter shaft 48 have been completely withdrawn from the reducing template 38 and removed from the stylet 46. The coating 60 that remains on the medical device 36 is complete and may be substantially uniform in thickness. Because the medical device 36 is initially submerged in the coating material 34, the medical device 36 is fully wetted by the coating material 34 ensuring a complete coating 60. Withdrawing the medical device 36 through the reducing template 38 ensures that the thickness of the coating 60 is predictable. Coatings produced by the apparatus are both predictable and repeatable.

In certain embodiments, the uniformity and coverage of the coating 60 can be improved by withdrawing the medical device 36 from the reducing template 38 with a twisting motion. Alternatively, this may be accomplished by twisting or rotating the reducing template 38 around the medical device 36 as the medical device 36 is being withdrawn. In another example embodiment, the uniformity and coverage of the coating 60 can be altered by changing the speed that the reducing template passes over the device.

**FIGS. 23A** and **23B** diagrammatically illustrate cross-sectional views of the reducing template 38, the catheter shaft 48, the stylet 46 and the medical device as the medical device is withdrawn through the reducing template 38 as shown in **FIG. 16A****.** **FIG. 23A** illustrates an embodiment in which the medical device 36 is in the form of a stent. An area defined by the cross-sectional inner profile 38a of the reducing template 38 is greater than an area defined by an outer profile 36a of the stent by a predetermined amount forming a gap area 50. In **FIG. 23A** the gap area 50 lies between the outer profile 36a of the stent and the cross-sectional inner profile 38a of the reducing template 38. The predetermined amount forming the gap area 50 is determined in part by a thickness of coating material 34 desired to remain on the medical device 36 after the medical device 36 is withdrawn from the reducing template 38. The reducing template 38 wipes excess coating material off of the medical device 36.

The shape, land length, and inner profile 38a of the reducing template ensure that the medical device will be uniformly coated. The thickness and uniformity of the coating on the medical device is determined by the shape and surface properties of the reducing template, the shape and surface properties of the medical device and the materials properties of the coating material. For a particular reducing template shape formed of a particular material, a particular medical device shape formed of another particular material, and a particular coating material, the cross-sectional inner profile of the reducing template is chosen relative to the outer profile of the medical device, in part, based on the desired coating thickness.

While the coating may be complete and the outer diameter of the coated device may be substantially uniform, the coating may not be uniformly thick if the medical device has an irregularly shaped surface. Because the reducing template removes excess coating material from the medical device, generally speaking, the larger the gap area, the more coating material remains on the medical device. The gap height along a radial line that runs through a point on the outer profile of the medical device partially determines the thickness of the coating at that point on the medical device. Line 51a connects a point on the outer profile 36a of the stent to a point on the cross-sectional inner profile 38a of the reducing template. The length of line 51a is the gap height at that point on the outer profile 36a of the stent. Line 51a connects to a point where the outer profile 36a is high, meaning that the gap height is relatively small. Line 51b connects to a point on the outer profile 36a of the stent that is about average in height meaning that the gap height is about average. Line 51c connects to a point on the outer profile 36a of the stent that is low meaning that that the gap height is relatively large. The relative thickness of the coating at a particular point on the outer profile 36a of the stent may be proportional to a gap height at that particular point. Because a gap height varies from point to point on the surface of the stent the coating thickness may vary from point to point on the surface of the stent. In addition to variations in gap area, variations in coating weight can be caused by the land of reducing template not being sufficiently wet out to lay down a consistent coating. Vapor lock can cause air bubbles to pass over the device as it is coated causing inconsistencies and should be avoided as well.

**FIG. 23B** illustrates the same view, but in this embodiment the medical device is a folded balloon 39 and the gap area 50 is shown between an outer profile 39a of the balloon and the cross-sectional inner profile 38a of the reducing template. Like the stent, the balloon 39 is not uniform in height meaning that a gap height varies for different points on the outer profile 39a of the balloon. At 33d the gap height is large, at 33e the gap height is about average and at 33f the gap height is small. An ideal average gap height varies with the coating material used, the medical device used, therapeutic agents used, and the details of treatment. For example, for some coating materials and some applications, an ideal average gap height is preferably between 0.0001 to 0.1 inches and more preferably between 0.001 to 0.01 inches. For some coating materials and some applications, the applicator can contact the device.

For a reducing template with a circular cross-section inner profile, the reducing template tends to remove coating material in a way that results in a substantially uniform outer diameter of the coated medical device after removal from the reducing template. However, the rheology of the coating material may cause flow of the coating material during and after removal of the medical device from the reducing template, which may alter the thickness distribution of the coating material. There are several rheological factors that can, along with the applicator design, effect coating weight consistency and distribution. Some of these rheological factors include viscosity, shear thinning, shear thickening, temperature dependent viscosity, thixotropic nature, Newtonian Vs. non Newtonian nature and creep. As the rheological properties of the coating change, the optimum internal diameter of the reducing template 38 may change. Lower viscosity materials may require a lower diameter reducing template 38 where higher viscosity materials may be able to tolerate a larger diameter reducing template 38.

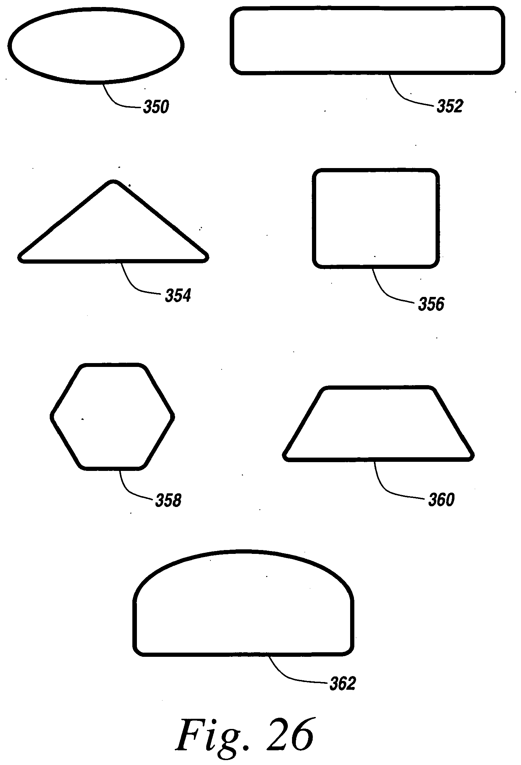

In the embodiment depicted in **FIGS. 15A-16B****,** the reducing template 38 has a circular cross-section that connects to a funnel or trumpet-like shaped portion of the sealed receptacle 32 at a distal end of the reducing template 38. Other suitable cross-sectional shapes include polygonal shapes such as hexagonal, octagonal, or the like as presented below with respect to **FIGS. 26-27C****.** Other possible shapes and configurations of the reducing template will be apparent to one skilled in the art given the benefit of this disclosure.

**FIGS. 17-20** illustrate additional embodiments of the present invention for coating a medical device according to different aspects of the present invention. **FIG. 17** diagrammatically illustrates a cross-sectional view of an apparatus 128 that includes an end cap 51, and includes a stylet 45b and a catheter cap 45a that are formed in one piece, but that can be optionally formed in separate pieces. The apparatus 128 also includes a sealed receptacle 108, a reducing template 138, a first seal 140, a medical device in the form of a stent 135 and a catheter shaft 148. A distal end 108b of the sealed receptacle can be formed of an end cap 51 that contains and preserves a coating material 134. The end cap 51 may be formed of any suitable material known in the art. As shown in this embodiment, the stylet 45b and the catheter cap 45a may be formed in one piece and fixed to the end cap 51. The end cap 51 is a second seal at the distal end 108b of the sealed receptacle.

**FIG. 18** diagrammatically illustrates a cross-sectional view of an apparatus 129 where a distal end 109b of the sealed receptacle is formed of a receptacle wall 133. The apparatus 129 includes a sealed receptacle 109, a reducing template 138, and a medical device in the form of a balloon 136. As shown in this embodiment a stylet 146 and a catheter cap 144 may be formed in one piece with the wall 133, or may be formed separately and fixed to the receptacle wall.

**FIG. 19** diagrammatically illustrates a cross-sectional view of an apparatus 130 where an end cap 150 is formed in one piece with a stylet 150b and a catheter cap150a. The apparatus also includes a sealed receptacle 110, a reducing template 138, a first seal 140, a catheter shaft 148, and a medical device in the form of a stent in combination with a balloon 137. In this embodiment, the end cap 150 forming a distal end 110b of the sealed receptacle contains and preserves the coating material 134. As shown in this embodiment, the end cap 150, the catheter cap 150a and the stylet 150b may all be formed in one piece, or in separate pieces.

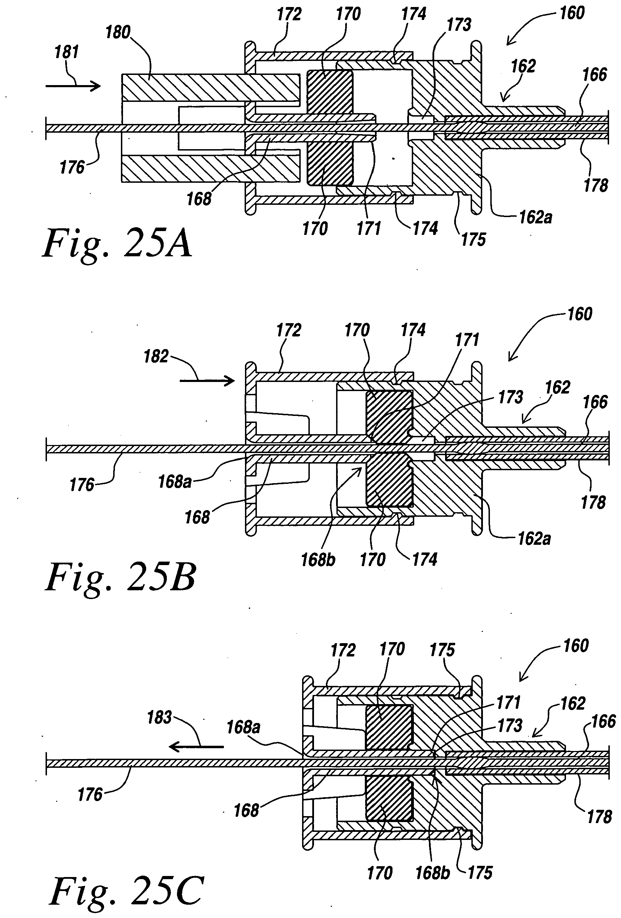

**FIG. 20** diagrammatically illustrates a cross-sectional view of an apparatus 131 with a first seal 141 disposed in the reducing template 138, a proximal end cover 152 and an end cap 151 substantially disposed within a receptacle wall 153. The apparatus 131 also includes a sealed receptacle 111, and a medical device in the form of a balloon 139. As shown in this embodiment, the apparatus 131 may or may not include a stylet or a catheter cap separate from the end cap 151. The end cap 151 may provide a seal to prevent coating material 134 from entering the interior of the catheter shaft 148 before use. Additionally, the end cap 151 may guide the motion of the catheter shaft 148 and the attached medical device, such as the balloon 139, when the catheter shaft 148 and the attached medical device, such as the balloon 139, are withdrawn from the reducing template 138. The end cap 151 may be disposed substantially within a receptacle wall 153. In this embodiment, the first seal 141, disposed at a proximal end 111a of the sealed receptacle and within the reducing template 138, must be completely removed or breached before the medical device, such as the balloon 139, is withdrawn through the reducing template 138. The proximal end cover 152 may be used to protect the sterility of the reducing template before use. One of ordinary skill in the art will appreciate that the first seal 141 can be positioned closer to the end cap 151 or that end of the sealed receptacle, such that the coating material 134 does not contact the reducing template (including the angled portion) while sealed, keeping the reducing template completely dry prior to breaking the seal of the sealed receptacle. This concept is further illustrated in **FIG. 25B** below, where the reducing template (as shown in that figure as #168) is on an opposite side of the seal from the coating material, thus also maintaining a dry state until use of the apparatus is required to coat the medical device.

**FIGS. 21A -21D** diagrammatically illustrate cross-sectional views of different embodiments of the distal end 32b of the sealed receptacle, according to aspects of the present invention. In **FIG. 21A** the distal end 32b of the sealed receptacle is formed of a receptacle wall 210. In **FIG. 21B** the distal end 32b of the sealed receptacle is formed of an end cap 212 that includes an o-ring seal 213. In **FIG. 21C** the distal end 32b of the sealed receptacle is formed of an end cap 214 disposed substantially within a receptacle wall 215. In **FIG. 21D****,** the distal end 32b of the sealed receptacle is formed by a crimp seal 216. While **FIGS. 15A-20** show particular embodiments of the distal end of the sealed receptacle in combination with particular other elements of the apparatus, one of skill in the art will recognize that any of the embodiments of the distal end may be combined with many different variations of the other elements of the apparatus and that the present invention is not limited to the combinations specifically depicted in the description.

A distal end of the sealed receptacle can provide a guide for guiding the motion of a catheter shaft as the medical device is withdrawn from the reducing template. Additionally, a distal end of the sealed receptacle can prevent coating material from entering a lumen of a catheter shaft before the apparatus is used. **FIGS. 21A-21D** present only a few embodiments of a distal end of the sealed receptacle; other embodiments of a distal end of the sealed receptacle configured to guide a catheter shaft and prevent coating material from entering the lumen of a catheter shaft will be apparent to one skilled in the art given the benefit of this disclosure.

**FIGS. 22A -22C** diagrammatically illustrate cross-sectional views of different embodiments of a proximal end of the sealed receptacle, according to aspects of the present invention. **FIG. 22A** shows an embodiment with a proximal end 32a of the sealed receptacle that includes a first seal 218 external to the reducing template 38 and disposed at a proximal end of the reducing template 38. **FIG. 22B** shows another embodiment with a proximal end 32a of the sealed receptacle that includes a first seal 220 disposed within a distal portion of the reducing template 38. Additionally, a shrink tubing proximal end cover 221 may be used to maintain the sterility of the reducing template 38. **FIG. 22C** shows yet another embodiment of a proximal end 32a of the sealed receptacle with a first seal in the form of a clamp 222 disposed about the distal portion of the reducing template 38. Additionally, a press fit proximal end cover 223 may be used to maintain the sterility of the reducing template 38. Examples of suitable materials and designs for the first seal include: shrink tubing or shrink film, a removable rubber grommet that can be applied or removed with a clip, a rubber grommet attached with a Thouy-Borst type fitting that slides in the direction of the distal end 32b of the sealed receptacle, clam shell fittings, snap fittings, an elastomeric tube acting as a tear away sleeve, two part fittings that may clamp a portion of the reducing template, or any other suitable materials and designs known in the art. While **FIGS. 15A-20** show particular embodiments of the proximal end of the sealed receptacle in combination with particular other elements of the apparatus, one of skill in the art will recognize that any embodiment of the proximal end of the sealed receptacle may be combined with many different variations of the other elements of the apparatus and that the present invention is not limited to the combinations specifically depicted herein.

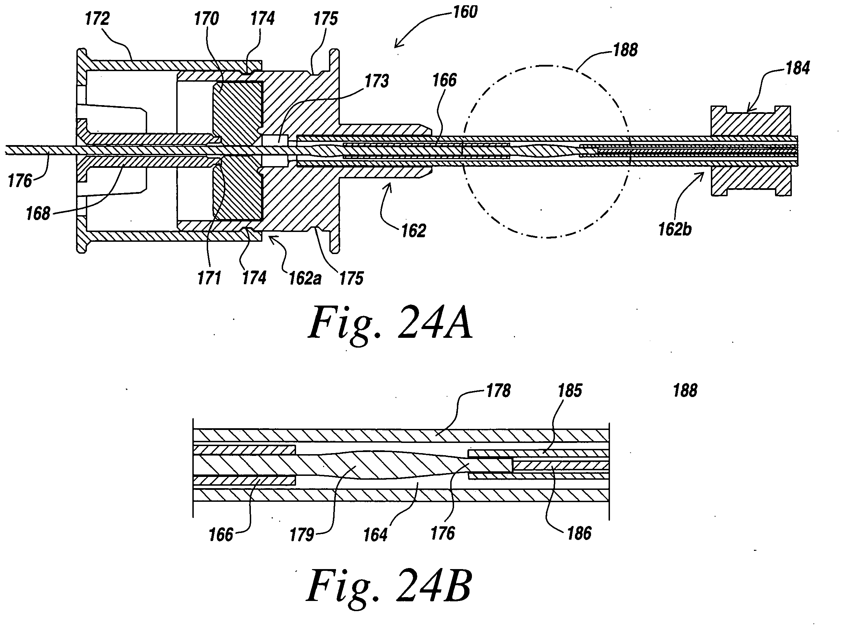

**FIG. 24A** depicts an exemplary embodiment of the present invention that includes an apparatus 160 having a first seal 170, a sleeve 172, and a seal breaching mechanism 171 that facilitate activation of the apparatus 160, according to aspects of the present invention. The apparatus 160 includes a sealed receptacle 162, with a proximal end 162a and a distal end 162b, that contains and preserves coating material 164. The sealed receptacle 162 is sealed by the first seal 170 at the proximal end 162a and sealed by a second crimp seal 184 at the distal end 162b. The second crimp seal 184 also fixes a catheter cap 185 and a stylet 186 to a receptacle tube 178 that forms a portion of the sealed receptacle 162 (see also **FIG. 24B****).** A medical device, such as a stent 166, is disposed within the sealed receptacle 162. The sleeve 172 is slidably coupled with the proximal end 162a of the sealed receptacle. A reducing template 168 is disposed within the sleeve 172 as shown. The sleeve 172 can further comprise the seal breaching mechanism 171 and a receiver 173 for receiving the seal breaching mechanism 171.

**FIG. 24B** depicts an enlarged view of a portion 188 of the apparatus 160 depicted in **FIG. 24A. FIG. 24B** shows the stent 166 and a catheter balloon cone 179 that prevents the stent 166 from sliding on the tip of the catheter shaft 176. The tip of the catheter shaft 176 is in contact with the catheter cap 185 and the stylet 186 to maintain the position of the catheter shaft 176 before use and to prevent coating material 164 from entering an inner lumen of the catheter shaft 176.

As shown in **FIG. 24A**, the apparatus 160 is in a pre-use configuration for shipping, storage, etc. **FIGS. 25A, 25B** and **25C** show a proximal portion of the apparatus 160 in various configurations: assembly, pre-use, and activation, respectively.

**FIG. 25A** diagrammatically illustrates the proximal end of the apparatus 160 as it is being assembled. In this configuration, the proximal end 162a of the receptacle 162 is not sealed by the first seal 170. This allows the catheter shaft 176 and the attached stent 166 to be inserted through the reducing template 168 into the portion of the receptacle 162 formed by the receptacle tube 178. After the catheter shaft 176 has been positioned, coating material 164 is introduced into the receptacle 162. After the medical device, such as the stent 166, and coating material 164 are in the receptacle 162, a plunger 180 can be used to move the first seal 170 in the direction of arrow 181, positioning the first seal 170 such that it makes sealing contact with the proximal end 162a of the sealed receptacle as shown in **FIG. 25B****.** The plunger 180 is only used for assembly and is removed after assembly.

**FIG. 25B** diagrammatically illustrates the proximal portion of the apparatus 160 after it has been assembled and is in a pre-use configuration for shipping, storage, etc. The first seal 170 makes sealing contact with the catheter shaft 176 and seals a proximal end 162a of the sealed receptacle. In this configuration, the stent 166 is immersed in the coating material 164, which is sealed and preserved in the sealed receptacle 162. The sleeve 172 and the proximal end 162a can be configurable relative to each other with one or more detents 174, 175 in a pre-use configuration, in an activation configuration, or both. As shown in **FIG. 25B****,** the sleeve 172 and the proximal end 162a are configured relative to each other in a pre-use configuration using detents 174. The sleeve 172 and the proximal end 162a are configurable relative to each other in an activation configuration with detents 175 (see also **FIG. 25C****).** When the stent 166 is needed, the apparatus 160 is activated by pushing the sleeve 172 in the direction of arrow 182 until the seal breaching mechanism 171 has completely breached the first seal 170 and is disposed in the receiver 173, forming a continuous inner lumen from the reducing template 168 to the receptacle 162, as shown in **FIG 25C****.**

**FIG. 25C** diagrammatically illustrates the proximal portion of the apparatus 160 in an activation configuration after it has been activated. The first seal 170 has been breached providing access to the stent 166 and the coating material 164. The seal breaching mechanism 171 and the receiver 173 allow the reducing template 168 to extend through the first seal 170 and form a continuous inner lumen with the receptacle 162 when the apparatus 160 has been activated. Although the small scale of the figure makes it difficult to observe, a lumen of the receptacle 162 has a larger inner diameter than an inner diameter of most of the reducing template 168. The inner diameter of the distal end of the reducing template 168b optionally gradually broadens to transition from the smaller inner diameter reducing template 168 to the larger inner diameter receptacle lumen. A proximal end 168a of the reducing template 168 optionally necks down to a diameter less than at least a portion of the reducing template 168.

In use, the catheter shaft 176 with the attached stent 166 is withdrawn from the apparatus 160 through the reducing template 168 in the direction of arrow 183. The reducing template 168 removes excess coating material 164 from the stent 166 resulting in a predictable, repeatable and substantially uniform coating on the stent 166. Although **FIG. 24** shows the proximal portion of the apparatus 160 including the first seal 170, sleeve 172, and seal breaching mechanism 171, in combination with a particular embodiment of a distal end of the apparatus 160, one of skill in the art will recognize that this embodiment of the proximal end of the apparatus 160 may be combined with many different embodiments of the distal end of the apparatus according to aspects of the present invention.

**FIGS. 15A -25C** illustrate some representative embodiments of the apparatus of the present invention. The various elements of the apparatus (ie. medical device, sealed receptacle, reducing template, first seal, second seal, sleeve, seal breaching mechanism, catheter, catheter cap, stylet, crimp seal, end cap, etc.) may be combined in combinations that are within the scope of the present invention, but are not specifically depicted in this specification due to the practical impossibility of depicting all possible combinations. In addition, the embodiments illustrated, and equivalents thereof, can be incorporated into a kit for providing a coated medical device. The kit primarily incorporating the apparatus of the present invention as described herein, in addition to instructions for use, as would be understood by those of ordinary skill in the art.

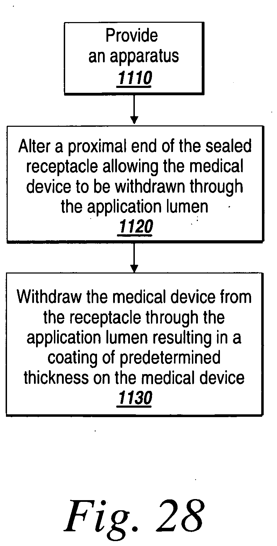

**FIG. 28** illustrates an exemplary method of making a coated medical device, according to aspects of the present invention. The method will be described, solely for illustrative purposes, with respect to the apparatus depicted in **FIGS. 15A-16B** and **23A.** One of skill in the art will recognize that many different embodiments of the apparatus could be used with the exemplary method. The process involves providing an apparatus 30 (step 1110). The apparatus 30 has a sealed receptacle 32 that contains and preserves a coating material 34. The sealed receptacle 32 has a proximal end 32a and a distal end 32b. A medical device 36 has an outer profile 36a. The medical device 36 is disposed and sealed within the sealed receptacle 32 and immersed in the coating material 34. The apparatus 30 also includes a reducing template 38 with a cross-sectional inner profile 38a, adapted to wipe excess coating material from the medical device 36. An area defined by the cross-sectional inner profile 38a of the reducing template is greater than an area defined by the outer profile 36a of the medical device by a predetermined amount forming a gap area 50. The predetermined amount forming the gap area 50 is determined at least in part by a thickness of coating material 34 desired to remain on the medical device 36 subsequent to movement of the medical device 36 through the reducing template 38 and out of the sealed receptacle 32.

The proximal end 32a of the sealed receptacle is altered allowing the medical device 36 to be withdrawn through the reducing template 38 (step 1120). Altering a proximal end of the sealed receptacle may include removing or physically altering a first seal 40 to allow for removal of the medical device 36. This alteration may result in the sealed receptacle 32 no longer being sealed.

The medical device 36 is withdrawn through the reducing template 38 resulting in a coating 60 of predetermined thickness on the medical device (step 1130). As discussed above, the medical device 36 is fully wetted by the coating material 34 because the medical device 36 is initially immersed in the coating material 34 resulting in a complete coating. As the medical device 36 is withdrawn from the reducing template 38, excess coating material 34 is removed from the medical device resulting in a substantially uniform coating with predetermined coating thickness. The medical device 36 may be rotated relative to the reducing template 38 about an axis along the reducing template 38 while being withdrawn.

One of ordinary skill in the art will appreciate that this method of application of a coating to a medical device can have a number of different variations falling within the process described. Depending on the particular application, the medical device 36 with the coating 60 applied thereon can be implanted immediately after the coating 60 is applied, or additional steps such as curing, sterilization, and removal of solvent can be applied to further prepare the medical device 36 with a coating 60. Furthermore, if the coating 60 includes a therapeutic agent that requires some form of activation (such as UV light), such actions can be implemented accordingly.

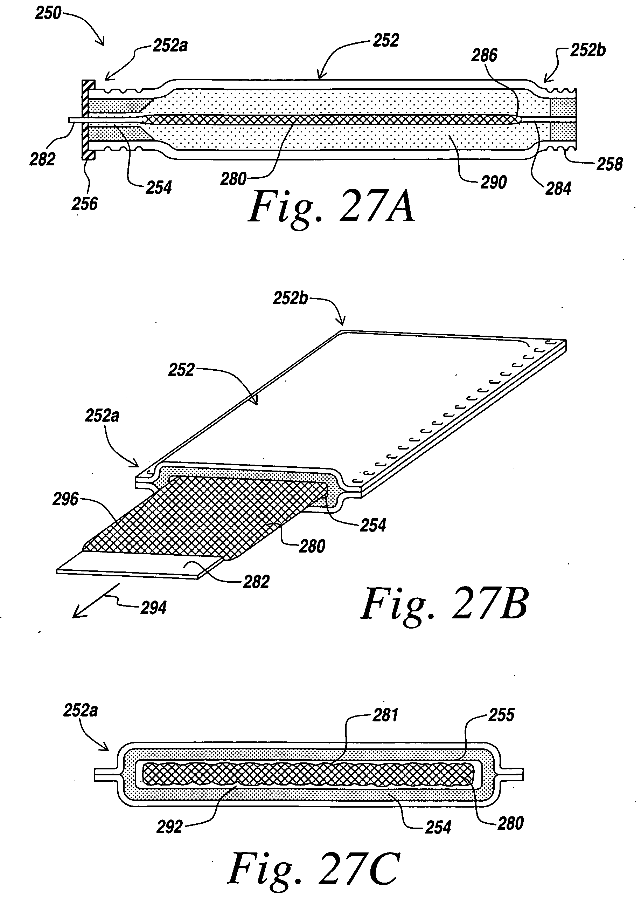

Although the medical devices depicted in the previous figures are substantially cylindrical in shape, each with a substantially circular outer profile, medical devices to be coated may have different outer profile shapes. Some examples of possible cross-sectional inner profile shapes are diagrammatically illustrated in **FIG. 26****.** Cross-sectional inner profile shapes can be substantially circular (see also **FIGS. 23A, 23B)** or substantially elliptical 350. Cross sectional inner profile shapes can be substantially polygonal, i.e. rectangular 352, triangular 354, square 356, hexagonal 358, trapezoidal 360, etc. Cross-sectional inner profile shapes can be substantially irregular 362. An example of an apparatus with a rectangular cross-sectional inner profile of the reducing template is depicted in **FIGS 27A** to **27C****.**

An apparatus 250 for coating a surgical mesh 280 with a coating material 290 is depicted in **FIGS. 27A** to **27C****,** according to aspects of the present invention. Apparatus 250 is suited to coat a medical device with a sheet-like or substantially planar shape. A sealed receptacle 252 is sealed by a removable first seal 256 at a proximal end 252a and sealed by a crimp seal 258 at a distal end 252b. The apparatus includes a reducing template 254. As can be seen in **FIG. 27C****,** which diagrammatically illustrates a cross-sectional view along the reducing template 254, the reducing template 254 has a cross-sectional inner profile 255 that is shaped like a rectangle with rounded corners. (The roughness of a cross-sectional outer profile 281 of the mesh is exaggerated for illustrative purposes). Between the rectangular inner profile 255 of the reducing template and the substantially rectangular outer profile 281 of the mesh lies a gap area 292. At one end the mesh 280 is connected to the distal end 252b of the sealed receptacle by a distal support 284. The mesh 280 and the distal support 284 are configured in a way that allows the mesh 280 to disconnect from the distal end 252b of the sealed receptacle when the apparatus 250 is activated and the mesh 280 is pulled through the reducing template 254. The mesh 280 can be connected to the distal support 284 by a weak seam 286 that will fail when the mesh 280 is pulled after activation of the apparatus 250. An opposite end of the mesh 280 is connected to an access tab 282. After the first seal 256 is breached, pulling on the access tab 282 in the direction of arrow 294 causes the mesh 280 to separate from the distal support 284. Further pulling of the access tab 282 causes the mesh 280 to be drawn through the reducing template 254, wiping off excess coating material 290, and resulting in a complete layer 296 of coating material on the mesh 280. Other medical devices with substantially planar shapes including films, patches and grafts, as well as non-cylindrical, non-planar shaped medical devices, fall within the scope of the present invention.

The application of the coating to the medical device can take place in a manufacturing-type facility and subsequently shipped and/or stored for later use. Alternatively, the coating can be applied to the medical device just prior to insertion or implantation in the patient. The medical device may undergo surface treatments before being immersed in the coating material in the sealed receptacle. The process utilized to prepare the medical device will vary according to the particular embodiment desired. In the case of the coating being applied immediately prior to use, the apparatus can be sterilized and sealed in packaging in a manufacturing facility. The apparatus can be stored at a medical facility until needed. The sealed receptacle protects the coating material from degrading before the apparatus is used. When needed, medical personal can remove sterile packaging and coat the medical device immediately prior to use.

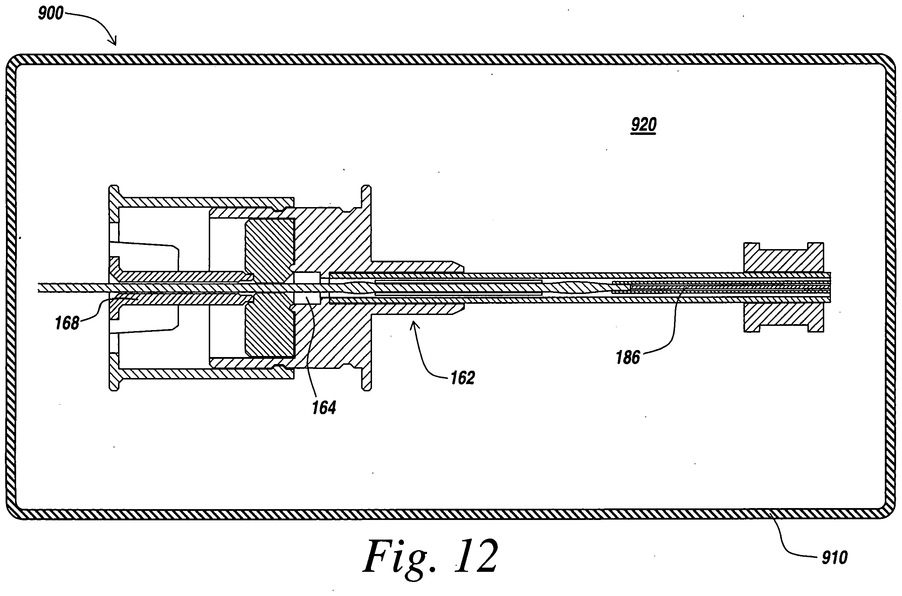

**FIG. 12** illustrates another embodiment of the coating device that includes an outer container 910 that preserves sterilization and further preserves the coating material from degradation. The apparatus 900 depicted in **FIG. 12** includes all of the elements of the apparatus 160 depicted in **Fig. 24A****.** The apparatus 900 further includes the outer container 910, and an inert gas 920 as described. The outer container 910 contains all other elements of the apparatus 900 and maintains the sterility of everything that it contains until the apparatus is used. The outer container 910 is filled with and contains an inert gas 920 that further preserves the coating material 164 until the apparatus is used. One of ordinary skill in the art will appreciate that a plurality of different embodiments as disclosed and described herein are suitable for including in an apparatus 900 that includes an outer container 910 for maintaining a sealable and sterilizable environment. As such, the embodiment shown in FIG. 12 is not limited to the specific medical device and/or apparatus including medical device, illustrated.

## Claims

1. An apparatus (30,128) for storing and coating a medical device (36,135), the apparatus comprising:
a sealed receptacle (32, 108) having a proximal end and a distal end, the receptacle containing a coating material (34, 134);
the medical device (36,135) having an outer profile, the medical device disposed and sealed within the sealed receptacle (32,108) and immersed in the coating material (34, 134), the sealed receptacle (32,108) configured to be unsealed or opened to enable the medical device (36,135) to pass through at a time of use; and
a reducing template (38, 138) having a cross-sectional inner profile, the reducing template adapted to receive the medical device (36, 135) and wipe excess of the coating material (34, 134) from the medical device;
wherein an area defined by the cross-sectional inner profile of the reducing template (38, 138) is greater than an area defined by the outer profile of the medical device (36,135) by a predetermined amount forming a gap area (50);
wherein the predetermined amount forming the gap area (50) is determined at least in part by a thickness of the coating material (34, 134) desired to remain on the medical device (36, 135) subsequent to movement of the medical device out of the sealed receptacle (32, 108) and through the reducing template (38, 138), wiping off excess coating material; and
wherein the sealed receptacle (32, 108) further comprises a catheter cap (44, 45a) disposed within the sealed receptacle (32, 108) and sealing a lumen of a catheter (48, 148).

2. The apparatus (30, 128) of claim 1, wherein the reducing template (38, 138) is:
coupled with the sealed receptacle (32, 108), disposed external to the sealed receptacle (32, 108), or disposed within the sealed receptacle (32, 108).

3. The apparatus (30, 128) of claim 1, wherein the sealed receptacle (32, 108) comprises the reducing template (38, 138), a proximal seal (40, 140) disposed at the proximal end of the sealed receptacle (32, 108), or comprises a distal seal (42, 51) disposed at the distal end of the sealed receptacle (32, 108).

4. The apparatus (131) of claim 1, wherein the apparatus further comprises a proximal end cover (152) disposed at a proximal end of the apparatus.

5. The apparatus (160) of claim 1, wherein the sealed receptacle (162) comprises a sleeve (172) coupled with the proximal end of the sealed receptacle in a slidable manner.

6. The apparatus (160) of claim 5, wherein the reducing template (168) is disposed within the sleeve (172).

7. The apparatus (160) of claim 5, wherein the sleeve (172) and the proximal end are configurable relative to each other with one or more detents (174, 175) in a pre-use configuration, in an activation configuration, or both.

8. The apparatus (160) of claim 7, wherein the sleeve (172) and the proximal end are slidable toward each other from the pre-use configuration to the activation configuration.

9. The apparatus (160) of claim 7, wherein the sleeve (172) further comprises a seal breaching mechanism (171) configured to breach a proximal seal (170) while the proximal end and the sleeve (172) are disposed in the activation configuration.

10. The (30, 128) apparatus of claim 1, wherein the sealed receptacle (32, 108) further comprises the catheter cap (44, 45a) disposed within the sealed receptacle (32, 108) and coupled with the sealed receptacle at the distal end.

11. The apparatus (30, 128) of claim 10, wherein the catheter cap (44, 45a) is permanently fixed to the distal end of the sealed receptacle (32, 108) by mechanical means, chemical means, thermal means, or any combination thereof.

12. The apparatus (30, 128) of claim 1, wherein the apparatus further comprises a stylet (46, 45b) partially disposed within the sealed receptacle (32, 108), coupled with the sealed receptacle (32, 108) at the distal end, and protruding from the sealed receptacle (32, 108) through the proximal end.

13. The apparatus (30, 128) of claim 12, wherein the stylet (46, 45b) is permanently fixed to the distal end of the sealed receptacle (32, 108) by mechanical means, chemical means, thermal means, or any combination thereof.

14. The apparatus (30, 128) of claim 1, wherein the catheter cap (44, 45a) is coupled with the sealed receptacle (32, 108) at the distal end.

15. The apparatus (128) of claim 1, wherein the sealed receptacle (108) comprises an end cap (51) disposed at the distal end of the sealed receptacle (108) in contact with a receptacle wall, wherein the end cap (51) includes a catheter cap (45a), a stylet (45b), or both.

16. The apparatus (30, 128) of claim 1, wherein the medical device (36, 135) is mounted on a catheter (48, 148).

17. The apparatus (30, 128) of claim 16 wherein the catheter (48, 148) protrudes from the sealed receptacle (32, 108) through the proximal end.

18. The apparatus (129) of claim 1, wherein the sealed receptacle (109) comprises a receptacle wall (133), and wherein the receptacle wall (133) comprises the catheter cap (144) disposed at the distal end of the sealed receptacle (109), a stylet (146) disposed at the distal end of the sealed receptacle (109), or both.

19. The apparatus (128) of claim 2, wherein the sealed receptacle (108) comprises an end cap (510) disposed at the distal end of the sealed receptacle (108) and in contact with a receptacle wall, wherein the end cap (51) includes the catheter cap (45a), a stylet (45b), or both.

20. The apparatus (30, 128) of claim 1, further comprising an outer container (910), wherein the sealed receptacle is disposed within the outer container (910) and the outer container (910) is adapted to preserve a sterile state of the sealed receptacle until use.

21. The apparatus (30, 128) of claim 20, further comprising inert gas (920) disposed within the outer container (910) to preserve the coating material.

22. The apparatus (30, 128) of claim 1, wherein the reducing template (38, 138) is elastic and the area defined by the cross-sectional inner profile of the reducing template (38, 138) is determined as the medical device moves through the reducing template (38, 138).

23. A method of coating a medical device (36, 135), comprising:
providing a storing and coating apparatus (30, 128) comprising:
a sealed receptacle (32, 108) having a proximal end and a distal end, the receptacle containing a coating material (34, 134);
the medical device (36, 135) having an outer profile, the medical device disposed and sealed within the sealed receptacle (32,108) and immersed in the coating material (34, 134), the sealed receptacle (32, 108) configured to be unsealed or opened to enable the medical device (36, 135) to pass through at a time of use; and
a reducing template (38, 138) having a cross-sectional inner profile, the reducing template adapted to receive the medical device (36, 135) and wipe excess of the coating material (34, 134) from the medical device;
wherein an area defined by the cross-sectional inner profile of the reducing template (38, 138) is greater than an area defined by the outer profile of the medical device (36, 135) by a predetermined amount forming a gap area (50); and
wherein the predetermined amount forming the gap area (50) is determined at least in part by a thickness of the coating material (34, 134) desired to remain on the medical device (36, 135) subsequent to movement of the medical device out of the sealed receptacle (32, 108) and through the reducing template (38, 138), wiping off excess of the coating material; and withdrawing the medical device (36, 135) through the reducing template (38, 138) causing the reducing template to regulate the thickness of the coating material (34, 134) formed on the medical device by wiping excess of the coating material from the medical device,
the method optionally further comprising unsealing or opening the sealed receptacle (32, 108) prior to withdrawing the medical device (36, 135) through the reducing template (38, 138).

24. A kit for coating a medical device (36, 135) comprising:
a coating material (34, 134);
the medical device (36, 135);
an apparatus (30, 128) for storing and coating the medical device (36, 135) as recited by claim 1;
and
instructions for use.

## Patentansprüche

1. Vorrichtung (30, 128) zum Aufbewahren und Beschichten einer medizinischen Vorrichtung (36, 135), wobei die Vorrichtung Folgendes umfasst:
ein versiegeltes Gefäß (32, 108), das ein proximales Ende und ein distales Ende aufweist, wobei das Gefäß ein Beschichtungsmaterial (34, 134) enthält;
die medizinische Vorrichtung (36, 135), die ein Außenprofil aufweist, wobei die medizinische Vorrichtung innerhalb des versiegelten Gefäßes (32, 108) angeordnet und versiegelt ist und in das Beschichtungsmaterial (34, 134) getaucht ist, wobei das versiegelte Gefäß (32, 108) konfiguriert ist, um entsiegelt oder geöffnet zu werden, um zu ermöglichen, dass die medizinische Vorrichtung (36, 135) zu einem Verwendungszeitpunkt hindurchtreten kann; und
eine Reduktionsschablone (38, 138), die ein Innenquerschnittsprofil aufweist, wobei die Reduktionsschablone geeignet ist, die medizinische Vorrichtung (36, 135) aufzunehmen und einen Überschuss des Beschichtungsmaterials (34, 134) von der medizinischen Vorrichtung abzustreifen;
wobei ein Raum, der vom Innenquerschnittsprofil der Reduktionsschablone (38, 138) abgegrenzt wird, um ein vorbestimmtes Maß, das einen Spaltraum (50) bildet, größer ist als ein Raum, der vom Außenprofil der medizinischen Vorrichtung (36, 135) abgegrenzt wird;
wobei das vorbestimmte Maß, das den Spaltraum (50) bildet, mindestens zum Teil durch eine Dicke des Beschichtungsmaterials (34, 134) bestimmt wird, die nach der Bewegung der medizinischen Vorrichtung aus dem versiegelten Gefäß (32, 108) und durch die Reduktionsschablone (38, 138), die überschüssiges Beschichtungsmaterial abstreift, auf der medizinischen Vorrichtung (36, 135) verbleiben soll; und
wobei das versiegelte Gefäß (32, 108) weiter einen Katheterverschluss (44, 45a) umfasst, der innerhalb des versiegelten Gefäßes (32, 108) angeordnet ist und ein Lumen eines Katheters (48, 148) versiegelt.

2. Vorrichtung (30, 128) nach Anspruch 1, wobei die Reduktionsschablone (38, 138):
mit dem versiegelten Gefäß (32, 108) gekoppelt ist, außerhalb des versiegelten Gefäßes (32, 108) angeordnet ist oder innerhalb des versiegelten Gefäßes (32, 108) angeordnet ist.

3. Vorrichtung (30, 128) nach Anspruch 1, wobei das versiegelte Gefäß (32, 108) die Reduktionsschablone (38, 138), ein proximales Siegel (40, 140), das am proximalen Ende des versiegelten Behälters (32, 108) angeordnet ist, umfasst oder ein distales Siegel (42, 51), das am distalen Ende des versiegelten Gefäßes (32, 108) angeordnet ist, umfasst.

4. Vorrichtung (131) nach Anspruch 1, wobei die Vorrichtung weiter eine proximale Endabdeckung (152) umfasst, die an einem proximalen Ende der Vorrichtung angeordnet ist.

5. Vorrichtung (160) nach Anspruch 1, wobei das versiegelte Gefäß (162) eine Hülle (172) umfasst, die an das proximale Ende des versiegelten Gefäßes verschiebbar gekoppelt ist.

6. Vorrichtung (160) nach Anspruch 5, wobei die Reduktionsschablone (168) innerhalb der Hülle (172) angeordnet ist.

7. Vorrichtung (160) nach Anspruch 5, wobei die Hülle (172) und das proximale Ende relativ zueinander mit einer oder mehreren Rasten (174, 175) in eine Vorverwendungskonfiguration, in eine Aktivierungskonfiguration oder in beide Konfigurationen konfigurierbar sind.

8. Vorrichtung (160) nach Anspruch 7, wobei die Hülle (172) und das proximale Ende von der Vorverwendungskonfiguration in die Aktivierungskonfiguration zueinander verschiebbar sind.

9. Vorrichtung (160) nach Anspruch 7, wobei die Hülle (172) weiter einen Mechanismus zum Aufbrechen des Siegels (171) umfasst, der konfiguriert ist, um ein proximales Siegel (170) aufzubrechen, während das proximale Ende und die Hülle (172) in der Aktivierungskonfiguration angeordnet sind.

10. Vorrichtung (30, 128) nach Anspruch 1, wobei das versiegelte Gefäß (32, 108) weiter den Katheterverschluss (44, 45a) umfasst, der innerhalb des versiegelten Gefäßes (32, 108) angeordnet ist und mit dem versiegelten Gefäß am distalen Ende gekoppelt ist.

11. Vorrichtung (30, 128) nach Anspruch 10, wobei der Katheterverschluss (44, 45a) dauerhaft am distalen Ende des versiegelten Gefäßes (32, 108) durch mechanische Mittel, chemische Mittel, thermische Mittel oder eine Kombination daraus fixiert ist.

12. Vorrichtung (30, 128) nach Anspruch 1, wobei die Vorrichtung weiter einen Führungsstab (46, 45b) umfasst, der teilweise innerhalb des versiegelten Gefäßes (32, 108) angeordnet ist, mit dem versiegelten Gefäß (32, 108) am distalen Ende gekoppelt ist und vom versiegelten Gefäß (32, 108) durch das proximale Ende hervorragt.

13. Vorrichtung (30, 128) nach Anspruch 12, wobei der Führungsstab (46, 45b) dauerhaft am distalen Ende des versiegelten Gefäßes (32, 108) durch mechanische Mittel, chemische Mittel, thermische Mittel oder eine Kombination daraus fixiert ist.

14. Vorrichtung (30, 128) nach Anspruch 1, wobei der Katheterverschluss (44, 45a) mit dem versiegelten Gefäß (32, 108) am distalen Ende gekoppelt ist.

15. Vorrichtung (128) nach Anspruch 1, wobei das versiegelte Gefäß (108) eine Abschlusskappe (51) umfasst, die am distalen Ende des versiegelten Gefäßes (108) in Kontakt mit einer Wand des Gefäßes angeordnet ist, wobei die Abschlusskappe (51) einen Katheterverschluss (45a), einen Führungsstab (45b) oder beides umfasst.

16. Vorrichtung (30, 128) nach Anspruch 1, wobei die medizinische Vorrichtung (36, 135) an einem Katheter (48, 148) angebracht ist.

17. Vorrichtung (30, 128) nach Anspruch 16, wobei der Katheter (48, 148) aus dem versiegelten Gefäß (32, 108) durch das proximale Ende hervorragt.

18. Vorrichtung (129) nach Anspruch 1, wobei das versiegelte Gefäß (109) eine Wand des Gefäßes (133) umfasst und wobei die Wand des Gefäßes (133) einen Katheterverschluss (144), der am distalen Ende des versiegelten Gefäßes (109) angeordnet ist, einen Führungsstab (146), der am distalen Ende des versiegelten Gefäßes (109) angeordnet ist, oder beides umfasst.

19. Vorrichtung (128) nach Anspruch 2, wobei das versiegelte Gefäß (108) eine Abschlusskappe (510) umfasst, die am distalen Ende des versiegelten Gefäßes (108) und in Kontakt mit einer Wand des Gefäßes angeordnet ist, wobei die Abschlusskappe (51) den Katheterverschluss (45a), einen Führungsstab (45b) oder beides umfasst.

20. Vorrichtung (30, 128) nach Anspruch 1, weiter umfassend einen Außenbehälter (910), wobei das versiegelte Gefäß innerhalb des Außenbehälters (910) angeordnet ist und der Außenbehälter (910) geeignet ist, einen sterilen Zustand des versiegelten Gefäßes bis zur Verwendung zu erhalten.

21. Vorrichtung (30, 128) nach Anspruch 20, weiter umfassend Inertgas (920), das innerhalb des Außenbehälters (910) angeordnet ist, um das Beschichtungsmaterial zu erhalten.

22. Vorrichtung (30, 128) nach Anspruch 1, wobei die Reduktionsschablone (38, 138) elastisch ist und der Raum, der durch das Innenquerschnittsprofil der Reduktionsschablone (38, 138) abgegrenzt wird, festgelegt wird, wenn sich die medizinische Vorrichtung durch die Reduktionsschablone (38, 138) bewegt.

23. Verfahren zum Beschichten einer medizinischen Vorrichtung (36, 135), umfassend:
Bereitstellen einer Aufbewahrungs- und Beschichtungsvorrichtung (30, 128), umfassend:
ein versiegeltes Gefäß (32, 108), das ein proximales Ende und ein distales Ende aufweist, wobei das Gefäß ein Beschichtungsmaterial (34, 134) enthält;
die medizinische Vorrichtung (36, 135), die ein Außenprofil aufweist, wobei die medizinische Vorrichtung innerhalb des versiegelten Gefäßes (32, 108) angeordnet und versiegelt ist und in das Beschichtungsmaterial (34, 134) getaucht ist, wobei das versiegelte Gefäß (32, 108) konfiguriert ist, um entsiegelt oder geöffnet zu werden, um zu ermöglichen, dass die medizinische Vorrichtung (36, 135) zu einem Verwendungszeitpunkt hindurchtreten kann; und
eine Reduktionsschablone (38, 138), die ein Innenquerschnittsprofil aufweist, wobei die Reduktionsschablone geeignet ist, die medizinische Vorrichtung (36, 135) aufzunehmen und einen Überschuss des Beschichtungsmaterials (34, 134) von der medizinischen Vorrichtung abzustreifen;
wobei ein Raum, der vom Innenquerschnittsprofil der Reduktionsschablone (38, 138) abgegrenzt wird, um ein vorbestimmtes Maß, das einen Spaltraum (50) bildet, größer ist als ein Raum, der vom Außenprofil der medizinischen Vorrichtung (36, 135) abgegrenzt wird; und
wobei das vorbestimmte Maß, das den Spaltraum (50) bildet, mindestens zum Teil durch eine Dicke des Beschichtungsmaterials (34, 134) bestimmt wird, die nach der Bewegung der medizinischen Vorrichtung aus dem versiegelten Gefäß (32, 108) und durch die Reduktionsschablone (38, 138), die überschüssiges Beschichtungsmaterial abstreift, auf der medizinischen Vorrichtung (36, 135) verbleiben soll; und
Entnehmen der medizinischen Vorrichtung (36, 135) durch die Reduktionsschablone (38, 138), wodurch bewirkt wird, dass die Reduktionsschablone die Dicke des Beschichtungsmaterials (34, 134), die auf der medizinischen Vorrichtung gebildet wurde, durch Abstreifen eines Überschusses des Beschichtungsmaterials von der medizinischen Vorrichtung reguliert,
wobei das Verfahren optional weiter das Entsiegeln oder Öffnen des versiegelten Gefäßes (32, 108) vor dem Entnehmen der medizinischen Vorrichtung (36, 135) durch die Reduktionsschablone (38, 138) umfasst.

24. Set zum Beschichten einer medizinischen Vorrichtung (36, 135), umfassend:
ein Beschichtungsmaterial (34, 134);
die medizinische Vorrichtung (36, 135);
eine Vorrichtung (30, 128) zum Aufbewahren und Beschichten der medizinischen Vorrichtung (36, 135) nach Anspruch 1;
und
eine Bedienungsanleitung.

## Revendications

1. Appareil (30, 128) de stockage et d'enduction d'un dispositif médical (36, 135), l'appareil comprenant :
un réceptacle scellé (32, 108) présentant une extrémité proximale et une extrémité distale, le réceptacle contenant un matériau d'enduction (34, 134) ;
le dispositif médical (36, 135) présentant un profil externe, le dispositif médical étant disposé et scellé dans le réceptacle scellé (32, 108) et immergé dans le matériau d'enduction (34, 134), le réceptacle scellé (32, 108) étant conçu pour être descellé ou ouvert afin de permettre le passage du dispositif médical (36, 135) lors de l'utilisation ; et
un gabarit réducteur (38, 138) présentant un profil interne transversal, le gabarit réducteur étant adapté pour recevoir le dispositif médical (36, 135) et essuyer l'excédent de matériau d'enduction (34, 134) présent sur le dispositif médical ;
dans lequel une zone définie par le profil interne transversal du gabarit réducteur (38, 138) est supérieure à une zone définie par le profil externe du dispositif médical (36, 135) d'une grandeur prédéterminée formant une zone interstitielle (50) ;
dans lequel la grandeur prédéterminée formant la zone interstitielle (50) est déterminée au moins en partie par une épaisseur du matériau d'enduction (34, 134) que l'on souhaite conserver sur le dispositif médical (36, 135) après le déplacement du dispositif médical hors du réceptacle scellé (32, 108) et à travers le gabarit réducteur (38, 138) essuyant l'excédent de matériau d'enduction ; et
dans lequel le réceptacle scellé (32, 108) comprend en outre un bouchon de cathéter (44, 45a) disposé à l'intérieur du réceptacle scellé (32, 108) et scellant la lumière d'un cathéter (48, 148).

2. Appareil (30, 128) selon la revendication 1, dans lequel le gabarit réducteur (38, 138) est :
couplé au réceptacle scellé (32, 108), disposé à l'extérieur du réceptacle scellé (32, 108), ou disposé à l'intérieur du réceptacle scellé (32, 108).

3. Appareil (30, 128) selon la revendication 1, dans lequel le réceptacle scellé (32, 108) comprend le gabarit réducteur (38, 138), un joint proximal (40, 140) disposé à l'extrémité proximale du réceptacle scellé (32, 108), ou un joint distal (42, 51) disposé à l'extrémité distale du réceptacle scellé (32, 108).

4. Appareil (131) selon la revendication 1, dans lequel l'appareil comprend en outre un couvercle d'extrémité proximal (152) disposé à une extrémité proximale de l'appareil.

5. Appareil (160) selon la revendication 1, dans lequel le réceptacle scellé (162) comprend un manchon (172) couplé de manière coulissante à l'extrémité proximale du réceptacle scellé.

6. Appareil (160) selon la revendication 5, dans lequel le gabarit réducteur (168) est disposé à l'intérieur du manchon (172) .

7. Appareil (160) selon la revendication 5, dans lequel le manchon (172) et l'extrémité proximale sont configurables l'un par rapport à l'autre avec un ou plusieurs cran(s) (174, 175) dans une configuration de pré-utilisation et/ou dans une configuration d'actionnement.

8. Appareil (160) selon la revendication 7, dans lequel le manchon (172) et l'extrémité proximale peuvent coulisser l'un vers l'autre de la configuration de pré-utilisation à la configuration d'actionnement.

9. Appareil (160) selon la revendication 7, dans lequel le manchon (172) comprend en outre un mécanisme de rupture de joint (171) conçu pour rompre un joint proximal (170) lorsque l'extrémité proximale et le manchon (172) sont disposés dans la configuration d'actionnement.

10. Appareil (30, 128) selon la revendication 1, dans lequel le réceptacle scellé (32, 108) comprend en outre le bouchon de cathéter (44, 45a) disposé à l'intérieur du réceptacle scellé (32, 108) et couplé au réceptacle scellé au niveau de l'extrémité distale.

11. Appareil (30, 128) selon la revendication 10, dans lequel le bouchon de cathéter (44, 45a) est fixé de manière permanente à l'extrémité distale du réceptacle scellé (32, 108) par des moyens mécaniques, chimiques, thermiques ou toute combinaison de ceux-ci.

12. Appareil (30, 128) selon la revendication 1, dans lequel l'appareil comprend en outre un stylet (46, 45b) partiellement disposé à l'intérieur du réceptacle scellé (32, 108), couplé avec le réceptacle scellé (32, 108) au niveau de l'extrémité distale, et dépassant du réceptacle scellé (32, 108) à travers l'extrémité proximale.

13. Appareil (30, 128) selon la revendication 12, dans lequel le stylet (46, 45b) est fixé de manière permanente à l'extrémité distale du réceptacle scellé (32, 108) par des moyens mécaniques, chimiques, thermiques ou toute combinaison de ceux-ci.

14. Appareil (30,128) selon la revendication 1, dans lequel le bouchon de cathéter (44,45a) est couplé au réceptacle scellé (32,108) au niveau de l'extrémité distale.

15. Appareil (128) selon la revendication 1, dans lequel le réceptacle scellé (108) comprend un bouchon d'extrémité (51) disposé à l'extrémité distale du réceptacle scellé (108) en contact avec une paroi du réceptacle, dans lequel le bouchon d'extrémité (51) comporte un bouchon de cathéter (45a) et/ou un stylet (45b).

16. Appareil (30,128) selon la revendication 1, dans lequel le dispositif médical (36,135) est monté sur un cathéter (48, 148).

17. Appareil (30, 128) selon la revendication 16, dans lequel le cathéter (48, 148) dépasse du réceptacle scellé (32, 108) à travers l'extrémité proximale.

18. Appareil (129) selon la revendication 1, dans lequel le réceptacle scellé (109) comprend une paroi de réceptacle (133), et dans lequel la paroi de réceptacle (133) comprend le bouchon de cathéter (144) disposé à l'extrémité distale du réceptacle scellé (109) et/ou un stylet (146) disposé à l'extrémité distale du réceptacle scellé (109).

19. Appareil (128) selon la revendication 2, dans lequel le réceptacle scellé (108) comprend un bouchon d'extrémité (510) disposé à l'extrémité distale du réceptacle scellé (108) et en contact avec une paroi du réceptacle, dans lequel le bouchon d'extrémité (51) comporte le bouchon de cathéter (45a) et/ou un stylet (45b).

20. Appareil (30, 128) selon la revendication 1, comprenant en outre un conteneur externe (910), dans lequel le rréceptacle scellé est disposé à l'intérieur du conteneur externe (910) et le conteneur externe (910) est adapté pour maintenir un état stérile du réceptacle scellé jusqu'à utilisation.

21. Appareil (30, 128) selon la revendication 20, comprenant en outre un gaz inerte (920) disposé à l'intérieur du conteneur externe (910) pour conserver le matériau d'enduction.

22. Appareil (30, 128) selon la revendication 1, dans lequel le gabarit réducteur (38, 138) est élastique et la zone définie par le profil interne transversal du gabarit réducteur (38, 138) est délimitée lors du passage du dispositif médical à travers le gabarit réducteur (38, 138).

23. Procédé d'enduction d'un dispositif médical (36, 135), consistant à :
fournir un appareil de stockage et d'enduction (30, 128) comprenant :
un réceptacle scellé (32, 108) présentant une extrémité proximale et une extrémité distale, le réceptacle contenant un matériau d'enduction (34, 134) ;
le dispositif médical (36,135) présentant un profil externe, le dispositif médical étant disposé et scellé à l'intérieur du réceptacle scellé (32, 108) et immergé dans le matériau d'enduction (34, 134), le réceptacle scellé (32, 108) étant conçu pour être descellé ou ouvert afin de permettre le passage du dispositif médical (36, 135) lors de l'utilisation ; et
un gabarit réducteur (38, 138) présentant un profil interne transversal, le gabarit réducteur étant adapté pour recevoir le dispositif médical (36, 135) et essuyer l'excédent de matériau d'enduction (34, 134) présent sur le dispositif médical ;
dans lequel une zone définie par le profil interne transversal du gabarit réducteur (38, 138) est supérieure à une zone définie par le profil externe du dispositif médical (36, 135) d'une grandeur prédéterminée formant une zone interstitielle (50) ; et
dans lequel la grandeur prédéterminée formant la zone interstitielle (50) est déterminée au moins en partie par une épaisseur du matériau d'enduction (34, 134) que l'on souhaite conserver sur le dispositif médical (36, 135) après le déplacement du dispositif médical hors du réceptacle scellé (32, 108) et à travers le gabarit réducteur (38, 138) essuyant l'excédent de matériau d'enduction ; et retirer le dispositif médical (36, 135) à travers le gabarit réducteur (38, 138), amenant le gabarit réducteur à ajuster l'épaisseur de matériau d'enduction (34, 134) formée sur le dispositif médical en essuyant l'excédent de matériau d'enduction présent sur le dispositif médical,
le procédé consistant en outre, facultativement, à desceller ou ouvrir le réceptacle scellé (32, 108) avant de retirer le dispositif médical (36, 135) à travers le gabarit réducteur (38, 138).

24. Kit d'enduction d'un dispositif médical (36, 135) comprenant :
un matériau d'enduction (34, 134) ; le dispositif médical (36, 135) ;
un appareil (30, 128) de stockage et d'enduction du dispositif médical (36, 135) selon la revendication 1 ;
et
un mode d'emploi.
